# EUROPEAN PATENT APPLICATION

(11) **EP 3 940 078 A1**
(43) Date of publication of application: **19.01.2022**
(21) Application number: 19916605.9
(22) Date of filing: 21.11.2019
(51) Int. Cl.: C12N 15/90, C12N 9/78, C12Q 1/34

(54) **OFF-TARGET SINGLE NUCLEOTIDE VARIANTS CAUSED BY SINGLE-BASE EDITING AND HIGH-SPECIFICITY OFF-TARGET-FREE SINGLE-BASE GENE EDITING TOOL**

(30) Priority: 28.02.2019 CN 201910153546; 09.06.2019 CN 201910494323
(71) Applicant: Center for Excellence in Brain Science and Intelligence Technology, Chinese Academy of Sciences, Xuhui District Shanghai 200031 (CN)
(72) Inventor: YANG, Hui, Shanghai 200031 (CN); ZUO, Erwei, Shanghai 200031 (CN)
(74) Representative: Potter Clarkson
(86) International application number: PCT/CN2019/119842
(87) International publication number: WO 2020/173150

(57) **Abstract**

Provided are a method for reducing the off-target effect of a single-base editor, and a method (GOTI) for analyzing the targeting effect of a gene editing tool or a gene editing operation.

Sequence Listing

<110>  Shanghai Institute of life sciences, Chinese Academy of Sciences

<120>  OFF-TARGET SINGLE NUCLEOTIDE VARIANTS CAUSED BY SINGLE-BASE EDITING
AND HIGH-SPECIFICITY OFF-TARGET-FREE SINGLE-BASE GENE EDITING TOOL

<130>  191650 PCWO

<150>  201910153546.3
<151>  2019-02-28

<150>  201910494323.3
<151>  2019-06-09

<160>  39

<170>  SIPOSequenceListing 1.0

<210>  1
<211>  1710
<212>  PRT
<213>  APOBEC1 wide type sequence (APOBEC1)

<400>  1
Met Ser Ser Glu Thr Gly Pro Val Ala Val Asp Pro Thr Leu Arg Arg
1               5                   10                  15
Arg Ile Glu Pro His Glu Phe Glu Val Phe Phe Asp Pro Arg Glu Leu
            20                  25                  30
Arg Lys Glu Thr Cys Leu Leu Tyr Glu Ile Asn Trp Gly Gly Arg His
        35                  40                  45
Ser Ile Trp Arg His Thr Ser Gln Asn Thr Asn Lys His Val Glu Val
    50                  55                  60
Asn Phe Ile Glu Lys Phe Thr Thr Glu Arg Tyr Phe Cys Pro Asn Thr
65                  70                  75                  80
Arg Cys Ser Ile Thr Trp Phe Leu Ser Trp Ser Pro Cys Gly Glu Cys
                85                  90                  95
Ser Arg Ala Ile Thr Glu Phe Leu Ser Arg Tyr Pro His Val Thr Leu
            100                 105                 110
Phe Ile Tyr Ile Ala Arg Leu Tyr His His Ala Asp Pro Arg Asn Arg
        115                 120                 125
Gln Gly Leu Arg Asp Leu Ile Ser Ser Gly Val Thr Ile Gln Ile Met
    130                 135                 140
Thr Glu Gln Glu Ser Gly Tyr Cys Trp Arg Asn Phe Val Asn Tyr Ser
145                 150                 155                 160
Pro Ser Asn Glu Ala His Trp Pro Arg Tyr Pro His Leu Trp Val Arg
                165                 170                 175
Leu Tyr Val Leu Glu Leu Tyr Cys Ile Ile Leu Gly Leu Pro Pro Cys
            180                 185                 190
Leu Asn Ile Leu Arg Arg Lys Gln Pro Gln Leu Thr Phe Phe Thr Ile
        195                 200                 205
Ala Leu Gln Ser Cys His Tyr Gln Arg Leu Pro Pro His Ile Leu Trp
    210                 215                 220
Ala Thr Gly Leu Lys Ser Gly Ser Glu Thr Pro Gly Thr Ser Glu Ser
225                 230                 235                 240
Ala Thr Pro Glu Ser Asp Lys Lys Tyr Ser Ile Gly Leu Ala Ile Gly
                245                 250                 255
Thr Asn Ser Val Gly Trp Ala Val Ile Thr Asp Glu Tyr Lys Val Pro
            260                 265                 270
Ser Lys Lys Phe Lys Val Leu Gly Asn Thr Asp Arg His Ser Ile Lys
        275                 280                 285
Lys Asn Leu Ile Gly Ala Leu Leu Phe Asp Ser Gly Glu Thr Ala Glu
    290                 295                 300

Ala Thr Arg Leu Lys Arg Thr Ala Arg Arg Arg Tyr Thr Arg Arg Lys
305                 310                 315                 320
Asn Arg Ile Cys Tyr Leu Gln Glu Ile Phe Ser Asn Glu Met Ala Lys
                325                 330                 335
Val Asp Asp Ser Phe Phe His Arg Leu Glu Glu Ser Phe Leu Val Glu
            340                 345                 350
Glu Asp Lys Lys His Glu Arg His Pro Ile Phe Gly Asn Ile Val Asp
        355                 360                 365
Glu Val Ala Tyr His Glu Lys Tyr Pro Thr Ile Tyr His Leu Arg Lys
    370                 375                 380
Lys Leu Val Asp Ser Thr Asp Lys Ala Asp Leu Arg Leu Ile Tyr Leu
385                 390                 395                 400
Ala Leu Ala His Met Ile Lys Phe Arg Gly His Phe Leu Ile Glu Gly
                405                 410                 415
Asp Leu Asn Pro Asp Asn Ser Asp Val Asp Lys Leu Phe Ile Gln Leu
            420                 425                 430
Val Gln Thr Tyr Asn Gln Leu Phe Glu Glu Asn Pro Ile Asn Ala Ser
        435                 440                 445
Gly Val Asp Ala Lys Ala Ile Leu Ser Ala Arg Leu Ser Lys Ser Arg
    450                 455                 460
Arg Leu Glu Asn Leu Ile Ala Gln Leu Pro Gly Glu Lys Lys Asn Gly
465                 470                 475                 480
Leu Phe Gly Asn Leu Ile Ala Leu Ser Leu Gly Leu Thr Pro Asn Phe
                485                 490                 495
Lys Ser Asn Phe Asp Leu Ala Glu Asp Ala Lys Leu Gln Leu Ser Lys
            500                 505                 510
Asp Thr Tyr Asp Asp Asp Leu Asp Asn Leu Leu Ala Gln Ile Gly Asp
        515                 520                 525
Gln Tyr Ala Asp Leu Phe Leu Ala Ala Lys Asn Leu Ser Asp Ala Ile
    530                 535                 540
Leu Leu Ser Asp Ile Leu Arg Val Asn Thr Glu Ile Thr Lys Ala Pro
545                 550                 555                 560
Leu Ser Ala Ser Met Ile Lys Arg Tyr Asp Glu His His Gln Asp Leu
                565                 570                 575
Thr Leu Leu Lys Ala Leu Val Arg Gln Gln Leu Pro Glu Lys Tyr Lys
            580                 585                 590
Glu Ile Phe Phe Asp Gln Ser Lys Asn Gly Tyr Ala Gly Tyr Ile Asp
        595                 600                 605
Gly Gly Ala Ser Gln Glu Glu Phe Tyr Lys Phe Ile Lys Pro Ile Leu
    610                 615                 620
Glu Lys Met Asp Gly Thr Glu Glu Leu Leu Val Lys Leu Asn Arg Glu
625                 630                 635                 640
Asp Leu Leu Arg Lys Gln Arg Thr Phe Asp Asn Gly Ser Ile Pro His
                645                 650                 655
Gln Ile His Leu Gly Glu Leu His Ala Ile Leu Arg Arg Gln Glu Asp
            660                 665                 670
Phe Tyr Pro Phe Leu Lys Asp Asn Arg Glu Lys Ile Glu Lys Ile Leu
        675                 680                 685
Thr Phe Arg Ile Pro Tyr Tyr Val Gly Pro Leu Ala Arg Gly Asn Ser
    690                 695                 700
Arg Phe Ala Trp Met Thr Arg Lys Ser Glu Glu Thr Ile Thr Pro Trp
705                 710                 715                 720
Asn Phe Glu Glu Val Val Asp Lys Gly Ala Ser Ala Gln Ser Phe Ile
                725                 730                 735
Glu Arg Met Thr Asn Phe Asp Lys Asn Leu Pro Asn Glu Lys Val Leu
            740                 745                 750
Pro Lys His Ser Leu Leu Tyr Glu Tyr Phe Thr Val Tyr Asn Glu Leu
        755                 760                 765
Thr Lys Val Lys Tyr Val Thr Glu Gly Met Arg Lys Pro Ala Phe Leu
    770                 775                 780
Ser Gly Glu Gln Lys Lys Ala Ile Val Asp Leu Leu Phe Lys Thr Asn
785                 790                 795                 800
Arg Lys Val Thr Val Lys Gln Leu Lys Glu Asp Tyr Phe Lys Lys Ile

                805                 810                 815
Glu Cys Phe Asp Ser Val Glu Ile Ser Gly Val Glu Asp Arg Phe Asn
            820                 825                 830
Ala Ser Leu Gly Thr Tyr His Asp Leu Leu Lys Ile Ile Lys Asp Lys
        835                 840                 845
Asp Phe Leu Asp Asn Glu Glu Asn Glu Asp Ile Leu Glu Asp Ile Val
    850                 855                 860
Leu Thr Leu Thr Leu Phe Glu Asp Arg Glu Met Ile Glu Glu Arg Leu
865                 870                 875                 880
Lys Thr Tyr Ala His Leu Phe Asp Asp Lys Val Met Lys Gln Leu Lys
                885                 890                 895
Arg Arg Arg Tyr Thr Gly Trp Gly Arg Leu Ser Arg Lys Leu Ile Asn
            900                 905                 910
Gly Ile Arg Asp Lys Gln Ser Gly Lys Thr Ile Leu Asp Phe Leu Lys
        915                 920                 925
Ser Asp Gly Phe Ala Asn Arg Asn Phe Met Gln Leu Ile His Asp Asp
    930                 935                 940
Ser Leu Thr Phe Lys Glu Asp Ile Gln Lys Ala Gln Val Ser Gly Gln
945                 950                 955                 960
Gly Asp Ser Leu His Glu His Ile Ala Asn Leu Ala Gly Ser Pro Ala
                965                 970                 975
Ile Lys Lys Gly Ile Leu Gln Thr Val Lys Val Val Asp Glu Leu Val
            980                 985                 990
Lys Val Met Gly Arg His Lys Pro Glu Asn Ile Val Ile Glu Met Ala
        995                 1000                1005
Arg Glu Asn Gln Thr Thr Gln Lys Gly Gln Lys Asn Ser Arg Glu Arg
    1010                1015                1020
Met Lys Arg Ile Glu Glu Gly Ile Lys Glu Leu Gly Ser Gln Ile Leu
1025                1030                1035                1040
Lys Glu His Pro Val Glu Asn Thr Gln Leu Gln Asn Glu Lys Leu Tyr
                1045                1050                1055
Leu Tyr Tyr Leu Gln Asn Gly Arg Asp Met Tyr Val Asp Gln Glu Leu
            1060                1065                1070
Asp Ile Asn Arg Leu Ser Asp Tyr Asp Val Asp His Ile Val Pro Gln
        1075                1080                1085
Ser Phe Leu Lys Asp Asp Ser Ile Asp Asn Lys Val Leu Thr Arg Ser
    1090                1095                1100
Asp Lys Asn Arg Gly Lys Ser Asp Asn Val Pro Ser Glu Glu Val Val
1105                1110                1115                1120
Lys Lys Met Lys Asn Tyr Trp Arg Gln Leu Leu Asn Ala Lys Leu Ile
                1125                1130                1135
Thr Gln Arg Lys Phe Asp Asn Leu Thr Lys Ala Glu Arg Gly Gly Leu
            1140                1145                1150
Ser Glu Leu Asp Lys Ala Gly Phe Ile Lys Arg Gln Leu Val Glu Thr
        1155                1160                1165
Arg Gln Ile Thr Lys His Val Ala Gln Ile Leu Asp Ser Arg Met Asn
    1170                1175                1180
Thr Lys Tyr Asp Glu Asn Asp Lys Leu Ile Arg Glu Val Lys Val Ile
1185                1190                1195                1200
Thr Leu Lys Ser Lys Leu Val Ser Asp Phe Arg Lys Asp Phe Gln Phe
                1205                1210                1215
Tyr Lys Val Arg Glu Ile Asn Asn Tyr His His Ala His Asp Ala Tyr
            1220                1225                1230
Leu Asn Ala Val Val Gly Thr Ala Leu Ile Lys Lys Tyr Pro Lys Leu
        1235                1240                1245
Glu Ser Glu Phe Val Tyr Gly Asp Tyr Lys Val Tyr Asp Val Arg Lys
    1250                1255                1260
Met Ile Ala Lys Ser Glu Gln Glu Ile Gly Lys Ala Thr Ala Lys Tyr
1265                1270                1275                1280
Phe Phe Tyr Ser Asn Ile Met Asn Phe Phe Lys Thr Glu Ile Thr Leu
                1285                1290                1295
Ala Asn Gly Glu Ile Arg Lys Arg Pro Leu Ile Glu Thr Asn Gly Glu
            1300                1305                1310

Thr Gly Glu Ile Val Trp Asp Lys Gly Arg Asp Phe Ala Thr Val Arg
        1315                1320                1325
Lys Val Leu Ser Met Pro Gln Val Asn Ile Val Lys Lys Thr Glu Val
    1330                1335                1340
Gln Thr Gly Gly Phe Ser Lys Glu Ser Ile Leu Pro Lys Arg Asn Ser
1345                1350                1355                1360
Asp Lys Leu Ile Ala Arg Lys Lys Asp Trp Asp Pro Lys Lys Tyr Gly
                1365                1370                1375
Gly Phe Asp Ser Pro Thr Val Ala Tyr Ser Val Leu Val Val Ala Lys
            1380                1385                1390
Val Glu Lys Gly Lys Ser Lys Lys Leu Lys Ser Val Lys Glu Leu Leu
        1395                1400                1405
Gly Ile Thr Ile Met Glu Arg Ser Ser Phe Glu Lys Asn Pro Ile Asp
    1410                1415                1420
Phe Leu Glu Ala Lys Gly Tyr Lys Glu Val Lys Lys Asp Leu Ile Ile
1425                1430                1435                1440
Lys Leu Pro Lys Tyr Ser Leu Phe Glu Leu Glu Asn Gly Arg Lys Arg
                1445                1450                1455
Met Leu Ala Ser Ala Gly Glu Leu Gln Lys Gly Asn Glu Leu Ala Leu
            1460                1465                1470
Pro Ser Lys Tyr Val Asn Phe Leu Tyr Leu Ala Ser His Tyr Glu Lys
        1475                1480                1485
Leu Lys Gly Ser Pro Glu Asp Asn Glu Gln Lys Gln Leu Phe Val Glu
    1490                1495                1500
Gln His Lys His Tyr Leu Asp Glu Ile Ile Glu Gln Ile Ser Glu Phe
1505                1510                1515                1520
Ser Lys Arg Val Ile Leu Ala Asp Ala Asn Leu Asp Lys Val Leu Ser
                1525                1530                1535
Ala Tyr Asn Lys His Arg Asp Lys Pro Ile Arg Glu Gln Ala Glu Asn
            1540                1545                1550
Ile Ile His Leu Phe Thr Leu Thr Asn Leu Gly Ala Pro Ala Ala Phe
        1555                1560                1565
Lys Tyr Phe Asp Thr Thr Ile Asp Arg Lys Arg Tyr Thr Ser Thr Lys
    1570                1575                1580
Glu Val Leu Asp Ala Thr Leu Ile His Gln Ser Ile Thr Gly Leu Tyr
1585                1590                1595                1600
Glu Thr Arg Ile Asp Leu Ser Gln Leu Gly Gly Asp Ser Gly Gly Ser
                1605                1610                1615
Thr Asn Leu Ser Asp Ile Ile Glu Lys Glu Thr Gly Lys Gln Leu Val
            1620                1625                1630
Ile Gln Glu Ser Ile Leu Met Leu Pro Glu Glu Val Glu Glu Val Ile
        1635                1640                1645
Gly Asn Lys Pro Glu Ser Asp Ile Leu Val His Thr Ala Tyr Asp Glu
    1650                1655                1660
Ser Thr Asp Glu Asn Val Met Leu Leu Thr Ser Asp Ala Pro Glu Tyr
1665                1670                1675                1680
Lys Pro Trp Ala Leu Val Ile Gln Asp Ser Asn Gly Glu Asn Lys Ile
                1685                1690                1695
Lys Met Leu Ser Gly Gly Ser Pro Lys Lys Lys Arg Lys Val
            1700                1705                1710

<210> 2
<211> 359
<212> PRT
<213> Cre protein sequence (Cre )

<400> 2
Met Pro Lys Lys Lys Arg Lys Val Ser Asn Leu Leu Thr Val His Gln
1               5                   10                  15
Asn Leu Pro Ala Leu Pro Val Asp Ala Thr Ser Asp Glu Val Arg Lys
            20                  25                  30
Asn Leu Met Asp Met Phe Arg Asp Arg Gln Ala Phe Ser Glu His Thr
        35                  40                  45

Trp Lys Met Leu Leu Ser Val Cys Arg Ser Trp Ala Ala Trp Cys Lys
    50                  55                  60
Leu Asn Asn Arg Lys Trp Phe Pro Ala Glu Pro Glu Asp Val Arg Asp
65                  70                  75                  80
Tyr Leu Leu Tyr Leu Gln Ala Arg Gly Leu Ala Val Lys Thr Ile Gln
                85                  90                  95
Gln His Leu Gly Gln Leu Asn Met Leu His Arg Arg Ser Gly Leu Pro
            100                 105                 110
Arg Pro Ser Asp Ser Asn Ala Val Ser Leu Val Met Arg Arg Ile Arg
        115                 120                 125
Lys Glu Asn Val Asp Ala Gly Glu Arg Ala Lys Gln Ala Leu Ala Phe
    130                 135                 140
Glu Arg Thr Asp Phe Asp Gln Val Arg Ser Leu Met Glu Asn Ser Asp
145                 150                 155                 160
Arg Cys Gln Asp Ile Arg Asn Leu Ala Phe Leu Gly Ile Ala Tyr Asn
                165                 170                 175
Thr Leu Leu Arg Ile Ala Glu Ile Ala Arg Ile Arg Val Lys Asp Ile
            180                 185                 190
Ser Arg Thr Asp Gly Gly Arg Met Leu Ile His Ile Gly Arg Thr Lys
        195                 200                 205
Thr Leu Val Ser Thr Ala Gly Val Glu Lys Ala Leu Ser Leu Gly Val
    210                 215                 220
Thr Lys Leu Val Glu Arg Trp Ile Ser Val Ser Gly Val Ala Asp Asp
225                 230                 235                 240
Pro Asn Asn Tyr Leu Phe Cys Arg Val Arg Lys Asn Gly Val Ala Ala
                245                 250                 255
Pro Ser Ala Thr Ser Gln Leu Ser Thr Arg Ala Leu Glu Gly Ile Phe
            260                 265                 270
Glu Ala Thr His Arg Leu Ile Tyr Gly Ala Lys Asp Asp Ser Gly Gln
        275                 280                 285
Arg Tyr Leu Ala Trp Ser Gly His Ser Ala Arg Val Gly Ala Ala Arg
    290                 295                 300
Asp Met Ala Arg Ala Gly Val Ser Ile Pro Glu Ile Met Gln Ala Gly
305                 310                 315                 320
Gly Trp Thr Asn Val Asn Ile Val Met Asn Tyr Ile Arg Asn Leu Asp
                325                 330                 335
Ser Glu Thr Gly Ala Met Val Arg Leu Leu Glu Asp Gly Asp Tyr Pro
            340                 345                 350
Tyr Asp Val Pro Asp Tyr Ala
        355

<210>  3
<211>  1423
<212>  PRT
<213>  Cas9 protein sequence (Cas9)

<400>  3
Met Asp Tyr Lys Asp His Asp Gly Asp Tyr Lys Asp His Asp Ile Asp
1               5                   10                  15
Tyr Lys Asp Asp Asp Asp Lys Met Ala Pro Lys Lys Lys Arg Lys Val
            20                  25                  30
Gly Ile His Gly Val Pro Ala Ala Asp Lys Lys Tyr Ser Ile Gly Leu
        35                  40                  45
Asp Ile Gly Thr Asn Ser Val Gly Trp Ala Val Ile Thr Asp Glu Tyr
    50                  55                  60
Lys Val Pro Ser Lys Lys Phe Lys Val Leu Gly Asn Thr Asp Arg His
65                  70                  75                  80
Ser Ile Lys Lys Asn Leu Ile Gly Ala Leu Leu Phe Asp Ser Gly Glu
                85                  90                  95
Thr Ala Glu Ala Thr Arg Leu Lys Arg Thr Ala Arg Arg Arg Tyr Thr
            100                 105                 110
Arg Arg Lys Asn Arg Ile Cys Tyr Leu Gln Glu Ile Phe Ser Asn Glu
        115                 120                 125

Met Ala Lys Val Asp Asp Ser Phe Phe His Arg Leu Glu Glu Ser Phe
    130                 135                 140
Leu Val Glu Glu Asp Lys Lys His Glu Arg His Pro Ile Phe Gly Asn
145                 150                 155                 160
Ile Val Asp Glu Val Ala Tyr His Glu Lys Tyr Pro Thr Ile Tyr His
                165                 170                 175
Leu Arg Lys Lys Leu Val Asp Ser Thr Asp Lys Ala Asp Leu Arg Leu
            180                 185                 190
Ile Tyr Leu Ala Leu Ala His Met Ile Lys Phe Arg Gly His Phe Leu
        195                 200                 205
Ile Glu Gly Asp Leu Asn Pro Asp Asn Ser Asp Val Asp Lys Leu Phe
    210                 215                 220
Ile Gln Leu Val Gln Thr Tyr Asn Gln Leu Phe Glu Glu Asn Pro Ile
225                 230                 235                 240
Asn Ala Ser Gly Val Asp Ala Lys Ala Ile Leu Ser Ala Arg Leu Ser
                245                 250                 255
Lys Ser Arg Arg Leu Glu Asn Leu Ile Ala Gln Leu Pro Gly Glu Lys
            260                 265                 270
Lys Asn Gly Leu Phe Gly Asn Leu Ile Ala Leu Ser Leu Gly Leu Thr
        275                 280                 285
Pro Asn Phe Lys Ser Asn Phe Asp Leu Ala Glu Asp Ala Lys Leu Gln
    290                 295                 300
Leu Ser Lys Asp Thr Tyr Asp Asp Asp Leu Asp Asn Leu Leu Ala Gln
305                 310                 315                 320
Ile Gly Asp Gln Tyr Ala Asp Leu Phe Leu Ala Ala Lys Asn Leu Ser
                325                 330                 335
Asp Ala Ile Leu Leu Ser Asp Ile Leu Arg Val Asn Thr Glu Ile Thr
            340                 345                 350
Lys Ala Pro Leu Ser Ala Ser Met Ile Lys Arg Tyr Asp Glu His His
        355                 360                 365
Gln Asp Leu Thr Leu Leu Lys Ala Leu Val Arg Gln Gln Leu Pro Glu
    370                 375                 380
Lys Tyr Lys Glu Ile Phe Phe Asp Gln Ser Lys Asn Gly Tyr Ala Gly
385                 390                 395                 400
Tyr Ile Asp Gly Gly Ala Ser Gln Glu Glu Phe Tyr Lys Phe Ile Lys
                405                 410                 415
Pro Ile Leu Glu Lys Met Asp Gly Thr Glu Glu Leu Leu Val Lys Leu
            420                 425                 430
Asn Arg Glu Asp Leu Leu Arg Lys Gln Arg Thr Phe Asp Asn Gly Ser
        435                 440                 445
Ile Pro His Gln Ile His Leu Gly Glu Leu His Ala Ile Leu Arg Arg
    450                 455                 460
Gln Glu Asp Phe Tyr Pro Phe Leu Lys Asp Asn Arg Glu Lys Ile Glu
465                 470                 475                 480
Lys Ile Leu Thr Phe Arg Ile Pro Tyr Tyr Val Gly Pro Leu Ala Arg
                485                 490                 495
Gly Asn Ser Arg Phe Ala Trp Met Thr Arg Lys Ser Glu Glu Thr Ile
            500                 505                 510
Thr Pro Trp Asn Phe Glu Glu Val Val Asp Lys Gly Ala Ser Ala Gln
        515                 520                 525
Ser Phe Ile Glu Arg Met Thr Asn Phe Asp Lys Asn Leu Pro Asn Glu
    530                 535                 540
Lys Val Leu Pro Lys His Ser Leu Leu Tyr Glu Tyr Phe Thr Val Tyr
545                 550                 555                 560
Asn Glu Leu Thr Lys Val Lys Tyr Val Thr Glu Gly Met Arg Lys Pro
                565                 570                 575
Ala Phe Leu Ser Gly Glu Gln Lys Lys Ala Ile Val Asp Leu Leu Phe
            580                 585                 590
Lys Thr Asn Arg Lys Val Thr Val Lys Gln Leu Lys Glu Asp Tyr Phe
        595                 600                 605
Lys Lys Ile Glu Cys Phe Asp Ser Val Glu Ile Ser Gly Val Glu Asp
    610                 615                 620
Arg Phe Asn Ala Ser Leu Gly Thr Tyr His Asp Leu Leu Lys Ile Ile

625                 630                 635                 640
Lys Asp Lys Asp Phe Leu Asp Asn Glu Glu Asn Glu Asp Ile Leu Glu
                645                 650                 655
Asp Ile Val Leu Thr Leu Thr Leu Phe Glu Asp Arg Glu Met Ile Glu
            660                 665                 670
Glu Arg Leu Lys Thr Tyr Ala His Leu Phe Asp Asp Lys Val Met Lys
        675                 680                 685
Gln Leu Lys Arg Arg Arg Tyr Thr Gly Trp Gly Arg Leu Ser Arg Lys
    690                 695                 700
Leu Ile Asn Gly Ile Arg Asp Lys Gln Ser Gly Lys Thr Ile Leu Asp
705                 710                 715                 720
Phe Leu Lys Ser Asp Gly Phe Ala Asn Arg Asn Phe Met Gln Leu Ile
                725                 730                 735
His Asp Asp Ser Leu Thr Phe Lys Glu Asp Ile Gln Lys Ala Gln Val
            740                 745                 750
Ser Gly Gln Gly Asp Ser Leu His Glu His Ile Ala Asn Leu Ala Gly
        755                 760                 765
Ser Pro Ala Ile Lys Lys Gly Ile Leu Gln Thr Val Lys Val Val Asp
    770                 775                 780
Glu Leu Val Lys Val Met Gly Arg His Lys Pro Glu Asn Ile Val Ile
785                 790                 795                 800
Glu Met Ala Arg Glu Asn Gln Thr Thr Gln Lys Gly Gln Lys Asn Ser
                805                 810                 815
Arg Glu Arg Met Lys Arg Ile Glu Glu Gly Ile Lys Glu Leu Gly Ser
            820                 825                 830
Gln Ile Leu Lys Glu His Pro Val Glu Asn Thr Gln Leu Gln Asn Glu
        835                 840                 845
Lys Leu Tyr Leu Tyr Tyr Leu Gln Asn Gly Arg Asp Met Tyr Val Asp
    850                 855                 860
Gln Glu Leu Asp Ile Asn Arg Leu Ser Asp Tyr Asp Val Asp His Ile
865                 870                 875                 880
Val Pro Gln Ser Phe Leu Lys Asp Asp Ser Ile Asp Asn Lys Val Leu
                885                 890                 895
Thr Arg Ser Asp Lys Asn Arg Gly Lys Ser Asp Asn Val Pro Ser Glu
            900                 905                 910
Glu Val Val Lys Lys Met Lys Asn Tyr Trp Arg Gln Leu Leu Asn Ala
        915                 920                 925
Lys Leu Ile Thr Gln Arg Lys Phe Asp Asn Leu Thr Lys Ala Glu Arg
    930                 935                 940
Gly Gly Leu Ser Glu Leu Asp Lys Ala Gly Phe Ile Lys Arg Gln Leu
945                 950                 955                 960
Val Glu Thr Arg Gln Ile Thr Lys His Val Ala Gln Ile Leu Asp Ser
                965                 970                 975
Arg Met Asn Thr Lys Tyr Asp Glu Asn Asp Lys Leu Ile Arg Glu Val
            980                 985                 990
Lys Val Ile Thr Leu Lys Ser Lys  Leu Val Ser Asp Phe  Arg Lys Asp
        995                 1000                1005
Phe Gln  Phe Tyr Lys Val Arg  Glu Ile Asn Asn Tyr  His His Ala His
    1010                1015                1020
Asp  Ala Tyr Leu Asn Ala  Val Val Gly Thr Ala  Leu Ile Lys Lys Tyr
1025                1030                1035                1040
Pro Lys Leu Glu Ser  Glu Phe Val Tyr Gly  Asp Tyr Lys Val Tyr  Asp
                1045                1050                1055
Val Arg Lys Met  Ile Ala Lys Ser Glu  Gln Glu Ile Gly Lys  Ala Thr
            1060                1065                1070
Ala Lys Tyr  Phe Phe Tyr Ser Asn  Ile Met Asn Phe Phe  Lys Thr Glu
        1075                1080                1085
Ile Thr  Leu Ala Asn Gly Glu  Ile Arg Lys Arg Pro  Leu Ile Glu Thr
    1090                1095                1100
Asn  Gly Glu Thr Gly Glu  Ile Val Trp Asp Lys  Gly Arg Asp Phe Ala
1105                1110                1115                1120
Thr Val Arg Lys Val  Leu Ser Met Pro Gln  Val Asn Ile Val Lys  Lys
                1125                1130                1135

Thr Glu Val Gln Thr Gly Gly Phe Ser Lys Glu Ser Ile Leu Pro Lys
            1140                1145                1150
Arg Asn Ser Asp Lys Leu Ile Ala Arg Lys Lys Asp Trp Asp Pro Lys
        1155                1160                1165
Lys Tyr Gly Gly Phe Asp Ser Pro Thr Val Ala Tyr Ser Val Leu Val
    1170                1175                1180
Val Ala Lys Val Glu Lys Gly Lys Ser Lys Lys Leu Lys Ser Val Lys
1185                1190                1195                1200
Glu Leu Leu Gly Ile Thr Ile Met Glu Arg Ser Ser Phe Glu Lys Asn
                1205                1210                1215
Pro Ile Asp Phe Leu Glu Ala Lys Gly Tyr Lys Glu Val Lys Lys Asp
            1220                1225                1230
Leu Ile Ile Lys Leu Pro Lys Tyr Ser Leu Phe Glu Leu Glu Asn Gly
        1235                1240                1245
Arg Lys Arg Met Leu Ala Ser Ala Gly Glu Leu Gln Lys Gly Asn Glu
    1250                1255                1260
Leu Ala Leu Pro Ser Lys Tyr Val Asn Phe Leu Tyr Leu Ala Ser His
1265                1270                1275                1280
Tyr Glu Lys Leu Lys Gly Ser Pro Glu Asp Asn Glu Gln Lys Gln Leu
                1285                1290                1295
Phe Val Glu Gln His Lys His Tyr Leu Asp Glu Ile Ile Glu Gln Ile
            1300                1305                1310
Ser Glu Phe Ser Lys Arg Val Ile Leu Ala Asp Ala Asn Leu Asp Lys
        1315                1320                1325
Val Leu Ser Ala Tyr Asn Lys His Arg Asp Lys Pro Ile Arg Glu Gln
    1330                1335                1340
Ala Glu Asn Ile Ile His Leu Phe Thr Leu Thr Asn Leu Gly Ala Pro
1345                1350                1355                1360
Ala Ala Phe Lys Tyr Phe Asp Thr Thr Ile Asp Arg Lys Arg Tyr Thr
                1365                1370                1375
Ser Thr Lys Glu Val Leu Asp Ala Thr Leu Ile His Gln Ser Ile Thr
            1380                1385                1390
Gly Leu Tyr Glu Thr Arg Ile Asp Leu Ser Gln Leu Gly Gly Asp Lys
        1395                1400                1405
Arg Pro Ala Ala Thr Lys Lys Ala Gly Gln Ala Lys Lys Lys Lys
    1410                1415                1420

<210> 4
<211> 1710
<212> PRT
<213> APOBEC1 mutant sequence (APOBEC1 mutant)

<400> 4
Met Ser Ser Glu Thr Gly Pro Val Ala Val Asp Pro Thr Leu Arg Arg
1               5                   10                  15
Arg Ile Glu Pro His Glu Phe Glu Val Phe Phe Asp Pro Arg Glu Leu
            20                  25                  30
Arg Lys Glu Thr Cys Leu Leu Tyr Glu Ile Asn Trp Gly Gly Arg His
        35                  40                  45
Ser Ile Trp Arg His Thr Ser Gln Asn Thr Asn Lys His Val Glu Val
    50                  55                  60
Asn Phe Ile Glu Lys Phe Thr Thr Glu Arg Tyr Phe Cys Pro Asn Thr
65                  70                  75                  80
Arg Cys Ser Ile Thr Trp Phe Leu Ser Trp Ser Pro Cys Gly Glu Cys
                85                  90                  95
Ser Arg Ala Ile Thr Glu Phe Leu Ser Arg Tyr Pro His Val Thr Leu
            100                 105                 110
Phe Ile Tyr Ile Ala Arg Leu Tyr His His Ala Asp Pro Glu Asn Arg
        115                 120                 125
Gln Gly Leu Arg Asp Leu Ile Ser Ser Gly Val Thr Ile Gln Ile Met
    130                 135                 140
Thr Glu Gln Glu Ser Gly Tyr Cys Trp Arg Asn Phe Val Asn Tyr Ser
145                 150                 155                 160

Pro Ser Asn Glu Ala His Trp Pro Arg Tyr Pro His Leu Trp Val Arg
                165                 170                 175
Leu Tyr Val Leu Glu Leu Tyr Cys Ile Ile Leu Gly Leu Pro Pro Cys
            180                 185                 190
Leu Asn Ile Leu Arg Arg Lys Gln Pro Gln Leu Thr Phe Phe Thr Ile
        195                 200                 205
Ala Leu Gln Ser Cys His Tyr Gln Arg Leu Pro Pro His Ile Leu Trp
    210                 215                 220
Ala Thr Gly Leu Lys Ser Gly Ser Glu Thr Pro Gly Thr Ser Glu Ser
225                 230                 235                 240
Ala Thr Pro Glu Ser Asp Lys Lys Tyr Ser Ile Gly Leu Ala Ile Gly
                245                 250                 255
Thr Asn Ser Val Gly Trp Ala Val Ile Thr Asp Glu Tyr Lys Val Pro
            260                 265                 270
Ser Lys Lys Phe Lys Val Leu Gly Asn Thr Asp Arg His Ser Ile Lys
        275                 280                 285
Lys Asn Leu Ile Gly Ala Leu Leu Phe Asp Ser Gly Glu Thr Ala Glu
    290                 295                 300
Ala Thr Arg Leu Lys Arg Thr Ala Arg Arg Arg Tyr Thr Arg Arg Lys
305                 310                 315                 320
Asn Arg Ile Cys Tyr Leu Gln Glu Ile Phe Ser Asn Glu Met Ala Lys
                325                 330                 335
Val Asp Asp Ser Phe Phe His Arg Leu Glu Glu Ser Phe Leu Val Glu
            340                 345                 350
Glu Asp Lys Lys His Glu Arg His Pro Ile Phe Gly Asn Ile Val Asp
        355                 360                 365
Glu Val Ala Tyr His Glu Lys Tyr Pro Thr Ile Tyr His Leu Arg Lys
    370                 375                 380
Lys Leu Val Asp Ser Thr Asp Lys Ala Asp Leu Arg Leu Ile Tyr Leu
385                 390                 395                 400
Ala Leu Ala His Met Ile Lys Phe Arg Gly His Phe Leu Ile Glu Gly
                405                 410                 415
Asp Leu Asn Pro Asp Asn Ser Asp Val Asp Lys Leu Phe Ile Gln Leu
            420                 425                 430
Val Gln Thr Tyr Asn Gln Leu Phe Glu Glu Asn Pro Ile Asn Ala Ser
        435                 440                 445
Gly Val Asp Ala Lys Ala Ile Leu Ser Ala Arg Leu Ser Lys Ser Arg
    450                 455                 460
Arg Leu Glu Asn Leu Ile Ala Gln Leu Pro Gly Glu Lys Lys Asn Gly
465                 470                 475                 480
Leu Phe Gly Asn Leu Ile Ala Leu Ser Leu Gly Leu Thr Pro Asn Phe
                485                 490                 495
Lys Ser Asn Phe Asp Leu Ala Glu Asp Ala Lys Leu Gln Leu Ser Lys
            500                 505                 510
Asp Thr Tyr Asp Asp Asp Leu Asp Asn Leu Leu Ala Gln Ile Gly Asp
        515                 520                 525
Gln Tyr Ala Asp Leu Phe Leu Ala Ala Lys Asn Leu Ser Asp Ala Ile
    530                 535                 540
Leu Leu Ser Asp Ile Leu Arg Val Asn Thr Glu Ile Thr Lys Ala Pro
545                 550                 555                 560
Leu Ser Ala Ser Met Ile Lys Arg Tyr Asp Glu His His Gln Asp Leu
                565                 570                 575
Thr Leu Leu Lys Ala Leu Val Arg Gln Gln Leu Pro Glu Lys Tyr Lys
            580                 585                 590
Glu Ile Phe Phe Asp Gln Ser Lys Asn Gly Tyr Ala Gly Tyr Ile Asp
        595                 600                 605
Gly Gly Ala Ser Gln Glu Glu Phe Tyr Lys Phe Ile Lys Pro Ile Leu
    610                 615                 620
Glu Lys Met Asp Gly Thr Glu Glu Leu Leu Val Lys Leu Asn Arg Glu
625                 630                 635                 640
Asp Leu Leu Arg Lys Gln Arg Thr Phe Asp Asn Gly Ser Ile Pro His
                645                 650                 655
Gln Ile His Leu Gly Glu Leu His Ala Ile Leu Arg Arg Gln Glu Asp

660                 665                 670
Phe Tyr Pro Phe Leu Lys Asp Asn Arg Glu Lys Ile Glu Lys Ile Leu
        675                 680                 685
Thr Phe Arg Ile Pro Tyr Tyr Val Gly Pro Leu Ala Arg Gly Asn Ser
    690                 695                 700
Arg Phe Ala Trp Met Thr Arg Lys Ser Glu Glu Thr Ile Thr Pro Trp
705                 710                 715                 720
Asn Phe Glu Glu Val Val Asp Lys Gly Ala Ser Ala Gln Ser Phe Ile
                725                 730                 735
Glu Arg Met Thr Asn Phe Asp Lys Asn Leu Pro Asn Glu Lys Val Leu
            740                 745                 750
Pro Lys His Ser Leu Leu Tyr Glu Tyr Phe Thr Val Tyr Asn Glu Leu
        755                 760                 765
Thr Lys Val Lys Tyr Val Thr Glu Gly Met Arg Lys Pro Ala Phe Leu
    770                 775                 780
Ser Gly Glu Gln Lys Lys Ala Ile Val Asp Leu Leu Phe Lys Thr Asn
785                 790                 795                 800
Arg Lys Val Thr Val Lys Gln Leu Lys Glu Asp Tyr Phe Lys Lys Ile
                805                 810                 815
Glu Cys Phe Asp Ser Val Glu Ile Ser Gly Val Glu Asp Arg Phe Asn
            820                 825                 830
Ala Ser Leu Gly Thr Tyr His Asp Leu Leu Lys Ile Ile Lys Asp Lys
        835                 840                 845
Asp Phe Leu Asp Asn Glu Glu Asn Glu Asp Ile Leu Glu Asp Ile Val
    850                 855                 860
Leu Thr Leu Thr Leu Phe Glu Asp Arg Glu Met Ile Glu Glu Arg Leu
865                 870                 875                 880
Lys Thr Tyr Ala His Leu Phe Asp Asp Lys Val Met Lys Gln Leu Lys
                885                 890                 895
Arg Arg Arg Tyr Thr Gly Trp Gly Arg Leu Ser Arg Lys Leu Ile Asn
            900                 905                 910
Gly Ile Arg Asp Lys Gln Ser Gly Lys Thr Ile Leu Asp Phe Leu Lys
        915                 920                 925
Ser Asp Gly Phe Ala Asn Arg Asn Phe Met Gln Leu Ile His Asp Asp
    930                 935                 940
Ser Leu Thr Phe Lys Glu Asp Ile Gln Lys Ala Gln Val Ser Gly Gln
945                 950                 955                 960
Gly Asp Ser Leu His Glu His Ile Ala Asn Leu Ala Gly Ser Pro Ala
                965                 970                 975
Ile Lys Lys Gly Ile Leu Gln Thr Val Lys Val Val Asp Glu Leu Val
            980                 985                 990
Lys Val Met Gly Arg His Lys Pro  Glu Asn Ile Val Ile  Glu Met Ala
        995                 1000                1005
Arg Glu  Asn Gln Thr Thr Gln  Lys Gly Gln Lys Asn  Ser Arg Glu Arg
    1010                1015                1020
Met  Lys Arg Ile Glu Glu  Gly Ile Lys Glu Leu  Gly Ser Gln Ile Leu
1025                1030                1035                1040
Lys Glu His Pro Val  Glu Asn Thr Gln Leu  Gln Asn Glu Lys Leu  Tyr
                1045                1050                1055
Leu Tyr Tyr Leu  Gln Asn Gly Arg Asp  Met Tyr Val Asp Gln  Glu Leu
            1060                1065                1070
Asp Ile Asn  Arg Leu Ser Asp Tyr  Asp Val Asp His Ile  Val Pro Gln
        1075                1080                1085
Ser Phe  Leu Lys Asp Asp Ser  Ile Asp Asn Lys Val  Leu Thr Arg Ser
    1090                1095                1100
Asp  Lys Asn Arg Gly Lys  Ser Asp Asn Val Pro  Ser Glu Glu Val Val
1105                1110                1115                1120
Lys Lys Met Lys Asn  Tyr Trp Arg Gln Leu  Leu Asn Ala Lys Leu  Ile
                1125                1130                1135
Thr Gln Arg Lys  Phe Asp Asn Leu Thr  Lys Ala Glu Arg Gly  Gly Leu
            1140                1145                1150
Ser Glu Leu  Asp Lys Ala Gly Phe  Ile Lys Arg Gln Leu  Val Glu Thr
        1155                1160                1165

Arg Gln Ile Thr Lys His Val Ala Gln Ile Leu Asp Ser Arg Met Asn
    1170                1175                1180
Thr Lys Tyr Asp Glu Asn Asp Lys Leu Ile Arg Glu Val Lys Val Ile
1185                1190                1195                1200
Thr Leu Lys Ser Lys Leu Val Ser Asp Phe Arg Lys Asp Phe Gln Phe
                1205                1210                1215
Tyr Lys Val Arg Glu Ile Asn Asn Tyr His His Ala His Asp Ala Tyr
            1220                1225                1230
Leu Asn Ala Val Val Gly Thr Ala Leu Ile Lys Lys Tyr Pro Lys Leu
        1235                1240                1245
Glu Ser Glu Phe Val Tyr Gly Asp Tyr Lys Val Tyr Asp Val Arg Lys
    1250                1255                1260
Met Ile Ala Lys Ser Glu Gln Glu Ile Gly Lys Ala Thr Ala Lys Tyr
1265                1270                1275                1280
Phe Phe Tyr Ser Asn Ile Met Asn Phe Phe Lys Thr Glu Ile Thr Leu
                1285                1290                1295
Ala Asn Gly Glu Ile Arg Lys Arg Pro Leu Ile Glu Thr Asn Gly Glu
            1300                1305                1310
Thr Gly Glu Ile Val Trp Asp Lys Gly Arg Asp Phe Ala Thr Val Arg
        1315                1320                1325
Lys Val Leu Ser Met Pro Gln Val Asn Ile Val Lys Lys Thr Glu Val
    1330                1335                1340
Gln Thr Gly Gly Phe Ser Lys Glu Ser Ile Leu Pro Lys Arg Asn Ser
1345                1350                1355                1360
Asp Lys Leu Ile Ala Arg Lys Lys Asp Trp Asp Pro Lys Lys Tyr Gly
                1365                1370                1375
Gly Phe Asp Ser Pro Thr Val Ala Tyr Ser Val Leu Val Val Ala Lys
            1380                1385                1390
Val Glu Lys Gly Lys Ser Lys Lys Leu Lys Ser Val Lys Glu Leu Leu
        1395                1400                1405
Gly Ile Thr Ile Met Glu Arg Ser Ser Phe Glu Lys Asn Pro Ile Asp
    1410                1415                1420
Phe Leu Glu Ala Lys Gly Tyr Lys Glu Val Lys Lys Asp Leu Ile Ile
1425                1430                1435                1440
Lys Leu Pro Lys Tyr Ser Leu Phe Glu Leu Glu Asn Gly Arg Lys Arg
                1445                1450                1455
Met Leu Ala Ser Ala Gly Glu Leu Gln Lys Gly Asn Glu Leu Ala Leu
            1460                1465                1470
Pro Ser Lys Tyr Val Asn Phe Leu Tyr Leu Ala Ser His Tyr Glu Lys
        1475                1480                1485
Leu Lys Gly Ser Pro Glu Asp Asn Glu Gln Lys Gln Leu Phe Val Glu
    1490                1495                1500
Gln His Lys His Tyr Leu Asp Glu Ile Ile Glu Gln Ile Ser Glu Phe
1505                1510                1515                1520
Ser Lys Arg Val Ile Leu Ala Asp Ala Asn Leu Asp Lys Val Leu Ser
                1525                1530                1535
Ala Tyr Asn Lys His Arg Asp Lys Pro Ile Arg Glu Gln Ala Glu Asn
            1540                1545                1550
Ile Ile His Leu Phe Thr Leu Thr Asn Leu Gly Ala Pro Ala Ala Phe
        1555                1560                1565
Lys Tyr Phe Asp Thr Thr Ile Asp Arg Lys Arg Tyr Thr Ser Thr Lys
    1570                1575                1580
Glu Val Leu Asp Ala Thr Leu Ile His Gln Ser Ile Thr Gly Leu Tyr
1585                1590                1595                1600
Glu Thr Arg Ile Asp Leu Ser Gln Leu Gly Gly Asp Ser Gly Gly Ser
                1605                1610                1615
Thr Asn Leu Ser Asp Ile Ile Glu Lys Glu Thr Gly Lys Gln Leu Val
            1620                1625                1630
Ile Gln Glu Ser Ile Leu Met Leu Pro Glu Glu Val Glu Glu Val Ile
        1635                1640                1645
Gly Asn Lys Pro Glu Ser Asp Ile Leu Val His Thr Ala Tyr Asp Glu
    1650                1655                1660
Ser Thr Asp Glu Asn Val Met Leu Leu Thr Ser Asp Ala Pro Glu Tyr

1665                1670                1675                1680
Lys Pro Trp Ala Leu  Val Ile Gln Asp Ser  Asn Gly Glu Asn Lys  Ile
                1685                1690                1695
Lys Met Leu Ser  Gly Gly Ser Pro Lys  Lys Lys Arg Lys Val
            1700                1705                1710

<210>  5
<211>  23
<212>  DNA
<213>  guide RNA(sgRNA)

<400>  5
gcgaaggcac cgccctcttt tgg                                              23

<210>  6
<211>  23
<212>  DNA
<213>  guide RNA(sgRNA)

<400>  6
ccagaagcca atgcacctat cgg                                              23

<210>  7
<211>  23
<212>  DNA
<213>  guide RNA(sgRNA)

<400>  7
tgcgaatacg cccacgcgat ggg                                              23

<210>  8
<211>  23
<212>  DNA
<213>  guide RNA(sgRNA)

<400>  8
ccaacctaag tagcagaaag tgg                                              23

<210>  9
<211>  23
<212>  DNA
<213>  guide RNA(sgRNA)

<400>  9
   gacctcagtt ccccttcaaa ggg                                             23

<210>  10
<211>  23
<212>  DNA
<213>  guide RNA(sgRNA)

<400>  10
ctgtgccaag gcagaaaccc tgg                                              23

<210>  11
<211>  23
<212>  DNA
<213>  guide RNA(sgRNA)

<400>  11
ccataacaga gactcttaca tgg                                              23

<210>  12

<211>  45
<212>  DNA
<213>  Primer

<400>  12
taatacgact cactataggg agacagatca cctttcctat caacc                    45

<210>  13
<211>  22
<212>  DNA
<213>  Primer

<400>  13
tcggtatttc cagcacactg ga                                             22

<210>  14
<211>  21
<212>  DNA
<213>  Primer

<400>  14
tccgcggccg ctaatacgac t                                              21

<210>  15
<211>  23
<212>  DNA
<213>  Primer

<400>  15
tggttctttc cgcctcagaa gcc                                            23

<210>  16
<211>  41
<212>  DNA
<213>  Primer

<400>  16
taatacgact cactataggg agatttcagg ttggaccggt g                        41

<210>  17
<211>  20
<212>  DNA
<213>  Primer

<400>  17
gacgtcagcg ttcgaattgc                                                20

<210>  18
<211>  22
<212>  DNA
<213>  Primer

<400>  18
gaggtctata taagcagagc tc                                             22

<210>  19
<211>  23
<212>  DNA
<213>  Primer

<400>  19
attaataact agcggccgct ccc                                            23

<210>  20
<211>  59
<212>  DNA
<213>  Primer

<400>  20
taatacgact cactataggg gcgaaggcac cgccctcttt gttttagagc tagaaatag          59

<210>  21
<211>  59
<212>  DNA
<213>  Primer

<400>  21
taatacgact cactataggg ccagaagcca atgcacctat gttttagagc tagaaatag          59

<210>  22
<211>  59
<212>  DNA
<213>  Primer

<400>  22
taatacgact cactataggg gacctcagtt ccccttcaaa gttttagagc tagaaatag          59

<210>  23
<211>  59
<212>  DNA
<213>  Primer

<400>  23
taatacgact cactataggg ctgtgccaag gcagaaaccc gttttagagc tagaaatag          59

<210>  24
<211>  59
<212>  DNA
<213>  Primer

<400>  24
taatacgact cactataggg ccataacaga gactcttaca gttttagagc tagaaatag          59

<210>  25
<211>  59
<212>  DNA
<213>  Primer

<400>  25
taatacgact cactataggg tgcgaatacg cccacgcgat gttttagagc tagaaatag          59

<210>  26
<211>  20
<212>  DNA
<213>  Primer

<400>  26
aaaagcaccg actcggtgcc                                                     20

<210>  27
<211>  21
<212>  DNA
<213>  Primer

<400>  27
gttatcctca cactacttct g                                                   21

<210>  28
<211>  21
<212>  DNA
<213>  Primer

<400>  28
gtaatcctac caagagtctc a                                                21

<210>  29
<211>  20
<212>  DNA
<213>  Primer

<400>  29
tcctcacact acttctgatg                                                  20

<210>  30
<211>  20
<212>  DNA
<213>  Primer

<400>  30
gtctcaagat ggaagatcac                                                  20

<210>  31
<211>  21
<212>  DNA
<213>  Primer

<400>  31
gttatcctca cactacttct g                                                21

<210>  32
<211>  21
<212>  DNA
<213>  Primer

<400>  32
gtaatcctac caagagtctc a                                                21

<210>  33
<211>  20
<212>  DNA
<213>  Primer

<400>  33
gtctgtgaca ctcattaacc                                                  20

<210>  34
<211>  21
<212>  DNA
<213>  Primer

<400>  34
cataggaggt gctaacaata c                                                21

<210>  35
<211>  20
<212>  DNA
<213>  Primer

<400>  35

gtattgcctt ctgtggagtt                                                    20

<210>  36
<211>  20
<212>  DNA
<213>  Primer

<400>  36
tgaaccaatc agtccttgtt                                                    20

<210>  37
<211>  1680
<212>  PRT
<213>  BE3-hA3A amino acid (BE3-hA3A)

<400>  37
Met Glu Ala Ser Pro Ala Ser Gly Pro Arg His Leu Met Asp Pro His
1               5                   10                  15
Ile Phe Thr Ser Asn Phe Asn Asn Gly Ile Gly Arg His Lys Thr Tyr
            20                  25                  30
Leu Cys Tyr Glu Val Glu Arg Leu Asp Asn Gly Thr Ser Val Lys Met
        35                  40                  45
Asp Gln His Arg Gly Phe Leu His Asn Gln Ala Lys Asn Leu Leu Cys
    50                  55                  60
Gly Phe Tyr Gly Arg His Ala Glu Leu Arg Phe Leu Asp Leu Val Pro
65                  70                  75                  80
Ser Leu Gln Leu Asp Pro Ala Gln Ile Tyr Arg Val Thr Trp Phe Ile
                85                  90                  95
Ser Trp Ser Pro Cys Phe Ser Trp Gly Cys Ala Gly Glu Val Arg Ala
            100                 105                 110
Phe Leu Gln Glu Asn Thr His Val Arg Leu Arg Ile Phe Ala Ala Arg
        115                 120                 125
Ile Tyr Asp Tyr Asp Pro Leu Tyr Lys Glu Ala Leu Gln Met Leu Arg
    130                 135                 140
Asp Ala Gly Ala Gln Val Ser Ile Met Thr Tyr Asp Glu Phe Lys His
145                 150                 155                 160
Cys Trp Asp Thr Phe Val Asp His Gln Gly Cys Pro Phe Gln Pro Trp
                165                 170                 175
Asp Gly Leu Asp Glu His Ser Gln Ala Leu Ser Gly Arg Leu Arg Ala
            180                 185                 190
Ile Leu Gln Asn Gln Gly Asn Ser Gly Ser Glu Thr Pro Gly Thr Ser
        195                 200                 205
Glu Ser Ala Thr Pro Glu Ser Asp Lys Lys Tyr Ser Ile Gly Leu Ala
    210                 215                 220
Ile Gly Thr Asn Ser Val Gly Trp Ala Val Ile Thr Asp Glu Tyr Lys
225                 230                 235                 240
Val Pro Ser Lys Lys Phe Lys Val Leu Gly Asn Thr Asp Arg His Ser
                245                 250                 255
Ile Lys Lys Asn Leu Ile Gly Ala Leu Leu Phe Asp Ser Gly Glu Thr
            260                 265                 270
Ala Glu Ala Thr Arg Leu Lys Arg Thr Ala Arg Arg Arg Tyr Thr Arg
        275                 280                 285
Arg Lys Asn Arg Ile Cys Tyr Leu Gln Glu Ile Phe Ser Asn Glu Met
    290                 295                 300
Ala Lys Val Asp Asp Ser Phe Phe His Arg Leu Glu Glu Ser Phe Leu
305                 310                 315                 320
Val Glu Glu Asp Lys Lys His Glu Arg His Pro Ile Phe Gly Asn Ile
                325                 330                 335
Val Asp Glu Val Ala Tyr His Glu Lys Tyr Pro Thr Ile Tyr His Leu
            340                 345                 350
Arg Lys Lys Leu Val Asp Ser Thr Asp Lys Ala Asp Leu Arg Leu Ile
        355                 360                 365
Tyr Leu Ala Leu Ala His Met Ile Lys Phe Arg Gly His Phe Leu Ile

370                 375                 380
Glu Gly Asp Leu Asn Pro Asp Asn Ser Asp Val Asp Lys Leu Phe Ile
385                 390                 395                 400
Gln Leu Val Gln Thr Tyr Asn Gln Leu Phe Glu Glu Asn Pro Ile Asn
                405                 410                 415
Ala Ser Gly Val Asp Ala Lys Ala Ile Leu Ser Ala Arg Leu Ser Lys
            420                 425                 430
Ser Arg Arg Leu Glu Asn Leu Ile Ala Gln Leu Pro Gly Glu Lys Lys
        435                 440                 445
Asn Gly Leu Phe Gly Asn Leu Ile Ala Leu Ser Leu Gly Leu Thr Pro
    450                 455                 460
Asn Phe Lys Ser Asn Phe Asp Leu Ala Glu Asp Ala Lys Leu Gln Leu
465                 470                 475                 480
Ser Lys Asp Thr Tyr Asp Asp Asp Leu Asp Asn Leu Leu Ala Gln Ile
                485                 490                 495
Gly Asp Gln Tyr Ala Asp Leu Phe Leu Ala Ala Lys Asn Leu Ser Asp
            500                 505                 510
Ala Ile Leu Leu Ser Asp Ile Leu Arg Val Asn Thr Glu Ile Thr Lys
        515                 520                 525
Ala Pro Leu Ser Ala Ser Met Ile Lys Arg Tyr Asp Glu His His Gln
    530                 535                 540
Asp Leu Thr Leu Leu Lys Ala Leu Val Arg Gln Gln Leu Pro Glu Lys
545                 550                 555                 560
Tyr Lys Glu Ile Phe Phe Asp Gln Ser Lys Asn Gly Tyr Ala Gly Tyr
                565                 570                 575
Ile Asp Gly Gly Ala Ser Gln Glu Glu Phe Tyr Lys Phe Ile Lys Pro
            580                 585                 590
Ile Leu Glu Lys Met Asp Gly Thr Glu Glu Leu Leu Val Lys Leu Asn
        595                 600                 605
Arg Glu Asp Leu Leu Arg Lys Gln Arg Thr Phe Asp Asn Gly Ser Ile
    610                 615                 620
Pro His Gln Ile His Leu Gly Glu Leu His Ala Ile Leu Arg Arg Gln
625                 630                 635                 640
Glu Asp Phe Tyr Pro Phe Leu Lys Asp Asn Arg Glu Lys Ile Glu Lys
                645                 650                 655
Ile Leu Thr Phe Arg Ile Pro Tyr Tyr Val Gly Pro Leu Ala Arg Gly
            660                 665                 670
Asn Ser Arg Phe Ala Trp Met Thr Arg Lys Ser Glu Glu Thr Ile Thr
        675                 680                 685
Pro Trp Asn Phe Glu Glu Val Val Asp Lys Gly Ala Ser Ala Gln Ser
    690                 695                 700
Phe Ile Glu Arg Met Thr Asn Phe Asp Lys Asn Leu Pro Asn Glu Lys
705                 710                 715                 720
Val Leu Pro Lys His Ser Leu Leu Tyr Glu Tyr Phe Thr Val Tyr Asn
                725                 730                 735
Glu Leu Thr Lys Val Lys Tyr Val Thr Glu Gly Met Arg Lys Pro Ala
            740                 745                 750
Phe Leu Ser Gly Glu Gln Lys Lys Ala Ile Val Asp Leu Leu Phe Lys
        755                 760                 765
Thr Asn Arg Lys Val Thr Val Lys Gln Leu Lys Glu Asp Tyr Phe Lys
    770                 775                 780
Lys Ile Glu Cys Phe Asp Ser Val Glu Ile Ser Gly Val Glu Asp Arg
785                 790                 795                 800
Phe Asn Ala Ser Leu Gly Thr Tyr His Asp Leu Leu Lys Ile Ile Lys
                805                 810                 815
Asp Lys Asp Phe Leu Asp Asn Glu Glu Asn Glu Asp Ile Leu Glu Asp
            820                 825                 830
Ile Val Leu Thr Leu Thr Leu Phe Glu Asp Arg Glu Met Ile Glu Glu
        835                 840                 845
Arg Leu Lys Thr Tyr Ala His Leu Phe Asp Asp Lys Val Met Lys Gln
    850                 855                 860
Leu Lys Arg Arg Arg Tyr Thr Gly Trp Gly Arg Leu Ser Arg Lys Leu
865                 870                 875                 880

Ile Asn Gly Ile Arg Asp Lys Gln Ser Gly Lys Thr Ile Leu Asp Phe
                885                 890                 895
Leu Lys Ser Asp Gly Phe Ala Asn Arg Asn Phe Met Gln Leu Ile His
            900                 905                 910
Asp Asp Ser Leu Thr Phe Lys Glu Asp Ile Gln Lys Ala Gln Val Ser
        915                 920                 925
Gly Gln Gly Asp Ser Leu His Glu His Ile Ala Asn Leu Ala Gly Ser
    930                 935                 940
Pro Ala Ile Lys Lys Gly Ile Leu Gln Thr Val Lys Val Val Asp Glu
945                 950                 955                 960
Leu Val Lys Val Met Gly Arg His Lys Pro Glu Asn Ile Val Ile Glu
                965                 970                 975
Met Ala Arg Glu Asn Gln Thr Thr Gln Lys Gly Gln Lys Asn Ser Arg
            980                 985                 990
Glu Arg Met Lys Arg Ile Glu Glu  Gly Ile Lys Glu Leu  Gly Ser Gln
        995                 1000                 1005
Ile Leu  Lys Glu His Pro Val  Glu Asn Thr Gln Leu  Gln Asn Glu Lys
    1010                 1015                 1020
Leu  Tyr Leu Tyr Tyr Leu  Gln Asn Gly Arg Asp  Met Tyr Val Asp Gln
1025                 1030                 1035                 1040
Glu Leu Asp Ile Asn  Arg Leu Ser Asp Tyr  Asp Val Asp His Ile  Val
                1045                 1050                 1055
Pro Gln Ser Phe  Leu Lys Asp Asp Ser  Ile Asp Asn Lys Val  Leu Thr
            1060                 1065                 1070
Arg Ser Asp  Lys Asn Arg Gly Lys  Ser Asp Asn Val Pro  Ser Glu Glu
        1075                 1080                 1085
Val Val  Lys Lys Met Lys Asn  Tyr Trp Arg Gln Leu  Leu Asn Ala Lys
    1090                 1095                 1100
Leu  Ile Thr Gln Arg Lys  Phe Asp Asn Leu Thr  Lys Ala Glu Arg Gly
1105                 1110                 1115                 1120
Gly Leu Ser Glu Leu  Asp Lys Ala Gly Phe  Ile Lys Arg Gln Leu  Val
                1125                 1130                 1135
Glu Thr Arg Gln  Ile Thr Lys His Val  Ala Gln Ile Leu Asp  Ser Arg
            1140                 1145                 1150
Met Asn Thr  Lys Tyr Asp Glu Asn  Asp Lys Leu Ile Arg  Glu Val Lys
        1155                 1160                 1165
Val Ile  Thr Leu Lys Ser Lys  Leu Val Ser Asp Phe  Arg Lys Asp Phe
    1170                 1175                 1180
Gln  Phe Tyr Lys Val Arg  Glu Ile Asn Asn Tyr  His His Ala His Asp
1185                 1190                 1195                 1200
Ala Tyr Leu Asn Ala  Val Val Gly Thr Ala  Leu Ile Lys Lys Tyr  Pro
                1205                 1210                 1215
Lys Leu Glu Ser  Glu Phe Val Tyr Gly  Asp Tyr Lys Val Tyr  Asp Val
            1220                 1225                 1230
Arg Lys Met  Ile Ala Lys Ser Glu  Gln Glu Ile Gly Lys  Ala Thr Ala
        1235                 1240                 1245
Lys Tyr  Phe Phe Tyr Ser Asn  Ile Met Asn Phe Phe  Lys Thr Glu Ile
    1250                 1255                 1260
Thr  Leu Ala Asn Gly Glu  Ile Arg Lys Arg Pro  Leu Ile Glu Thr Asn
1265                 1270                 1275                 1280
Gly Glu Thr Gly Glu  Ile Val Trp Asp Lys  Gly Arg Asp Phe Ala  Thr
                1285                 1290                 1295
Val Arg Lys Val  Leu Ser Met Pro Gln  Val Asn Ile Val Lys  Lys Thr
            1300                 1305                 1310
Glu Val Gln  Thr Gly Gly Phe Ser  Lys Glu Ser Ile Leu  Pro Lys Arg
        1315                 1320                 1325
Asn Ser  Asp Lys Leu Ile Ala  Arg Lys Lys Asp Trp  Asp Pro Lys Lys
    1330                 1335                 1340
Tyr  Gly Gly Phe Asp Ser  Pro Thr Val Ala Tyr  Ser Val Leu Val Val
1345                 1350                 1355                 1360
Ala Lys Val Glu Lys  Gly Lys Ser Lys Lys  Leu Lys Ser Val Lys  Glu
                1365                 1370                 1375
Leu Leu Gly Ile  Thr Ile Met Glu Arg  Ser Ser Phe Glu Lys  Asn Pro

1380                1385                1390
Ile Asp Phe Leu Glu Ala Lys Gly Tyr Lys Glu Val Lys Lys Asp Leu
        1395                1400                1405
Ile Ile Lys Leu Pro Lys Tyr Ser Leu Phe Glu Leu Glu Asn Gly Arg
    1410                1415                1420
Lys Arg Met Leu Ala Ser Ala Gly Glu Leu Gln Lys Gly Asn Glu Leu
1425                1430                1435                1440
Ala Leu Pro Ser Lys Tyr Val Asn Phe Leu Tyr Leu Ala Ser His Tyr
                1445                1450                1455
Glu Lys Leu Lys Gly Ser Pro Glu Asp Asn Glu Gln Lys Gln Leu Phe
            1460                1465                1470
Val Glu Gln His Lys His Tyr Leu Asp Glu Ile Ile Glu Gln Ile Ser
        1475                1480                1485
Glu Phe Ser Lys Arg Val Ile Leu Ala Asp Ala Asn Leu Asp Lys Val
    1490                1495                1500
Leu Ser Ala Tyr Asn Lys His Arg Asp Lys Pro Ile Arg Glu Gln Ala
1505                1510                1515                1520
Glu Asn Ile Ile His Leu Phe Thr Leu Thr Asn Leu Gly Ala Pro Ala
                1525                1530                1535
Ala Phe Lys Tyr Phe Asp Thr Thr Ile Asp Arg Lys Arg Tyr Thr Ser
            1540                1545                1550
Thr Lys Glu Val Leu Asp Ala Thr Leu Ile His Gln Ser Ile Thr Gly
        1555                1560                1565
Leu Tyr Glu Thr Arg Ile Asp Leu Ser Gln Leu Gly Gly Asp Ser Gly
    1570                1575                1580
Gly Ser Thr Asn Leu Ser Asp Ile Ile Glu Lys Glu Thr Gly Lys Gln
1585                1590                1595                1600
Leu Val Ile Gln Glu Ser Ile Leu Met Leu Pro Glu Glu Val Glu Glu
                1605                1610                1615
Val Ile Gly Asn Lys Pro Glu Ser Asp Ile Leu Val His Thr Ala Tyr
            1620                1625                1630
Asp Glu Ser Thr Asp Glu Asn Val Met Leu Leu Thr Ser Asp Ala Pro
        1635                1640                1645
Glu Tyr Lys Pro Trp Ala Leu Val Ile Gln Asp Ser Asn Gly Glu Asn
    1650                1655                1660
Lys Ile Lys Met Leu Ser Gly Gly Ser Pro Lys Lys Lys Arg Lys Val
1665                1670                1675                1680

<210>  38
<211>  16
<212>  PRT
<213>  linker

<400>  38
Ser Gly Ser Glu Thr Pro Gly Thr Ser Glu Ser Ala Thr Pro Glu Ser
1               5                   10                  15

<210>  39
<211>  4
<212>  PRT
<213>  linker

<400>  39
Ser Gly Gly Ser
1

## Description

### Technical field

The present invention belongs to the technical field of gene editing. More specifically, the present invention relates to non-targeted single-nucleotide mutations leaded by single-base editing. The present invention also relates to high-specific non-off-target single-nucleotide gene editing tools for avoiding such mutations.

### BACKGROUND OF DISCLOSURE

Genome editing technology has been highly valued since its inception. CRISPR is the abbreviation of clustered regularly interspaced short palindromic repeats, and Cas is the abbreviation of CRISPR associate protein. CRISPR/Cas was originally found in bacteria, and is used by bacteria as defense system to identify and destroy the invasion of bacteriophages and other pathogens. In the CRISPR/Cas9 system, the enzyme Cas9 cuts on the DNA target site. Cas9 together with sgRNA is called the Cas9-sgRNA system. CRISPR/Cas9 technology has been applied to disease model establishment, drug target screening, and is becoming a new generation of gene therapy methods.

Gene editing methods mediated by CRISPR/Cas9 and base editors have been developed, and have brought great hope for the treatment of genetic diseases caused by pathogenic mutations. Clinical applications based on CRISPR/Cas9 gene editing or base editing require comprehensive analysis of off-target effects to reduce the risk of harmful mutations. Although a variety of methods have been developed in the field to detect the off-target activity of genome-wide gene editing cells, including High-Throughput Genome-Wide Translocation Sequencing (HTGTS), Genome-wide Unbiased Indentification of DSBs Evaluated by Sequencing (GUIDE-seq) and Circularization for In vitro Reporting of Cleavage Effects by Sequencing (CIRCLE-seq). However, none of these methods can effectively detect single-nucleotide variants (SNVs). So far no method can effectively detect SNVs in this field.

Moreover, a defect of CRISPR/Cas9 lies in the low editing efficiency of homology-mediated repair. Those skilled in the art use a 16-base XTEN linker to link the cytidine deaminase APOBEC1 and dCas9 together to construct the first generation base editor (BE1). In order to increase editing efficiency in vivo, in addition to linking cytidine deaminase and dCas9, the second-generation base editor system (BE2) also fuses base excision repair inhibitor UGI to dCas9, and editing efficiency is increased three times, up to about 20%.

In order to further improve the efficiency of base editing, those skilled in the art replaced dCas9 with Cas9n to simulate mismatch repair, thereby constructing a third-generation base editor (BE3). BE3 creates a nick in the non-complementary DNA strand, and the cell uses the DNA strand containing uracil (U) as a template for repair, thereby replicating such base editing. Among a variety of target genes in human cell lines, BE3 system significantly improves the base editing efficiency, and its average indel (insertion-deletion) incidence is only 1.1%. For the tested target genes, these numbers show a huge improvement over Cas9-mediated HDR. The average HDR-mediated editing frequency is only 0.5%, and compared to previous single-base editing, more indels are observed. CRISPR base editing persists after multiple cell divisions, indicating that this method produces stable base editing. However, this BE3 system also affected by off-target editing.

Genome editing has great potential to treat genetic diseases induced by pathogenic mutations. Comprehensive analysis of off-target effects of gene editing is very necessary for its practicality. At the same time, the field still needs to find a solution for the off-target problem.

### SUMMARY OF DISCLOSURE

The purpose of the present invention is to study the phenomenon that single-base editing leads to non-targeted single-nucleotide mutations, and to provide a high-specific non-off-target single-base gene editing tool.

In the first aspect of the present invention, a method for reducing the off-target effect of a single-base editor is provided, including: modifying the cytosine deaminase in the single base editor system to weaken its binding to DNA.

In a preferred embodiment, the modification is to modify the DNA binding region of cytosine deaminase; preferably, the DNA binding region is a domain that binds to DNA (such as ssDNA).

In another preferred embodiment, the modification includes, but is not limited to: gene mutation, targeted blocking (such as blocking by binding proteins or antibodies, or blocking by competitive binding molecules), interference.

In another preferred embodiment, the single-base editor system is a BE3 gene editor system.

In another preferred embodiment, the DNA is single-stranded DNA (ssDNA) or double-stranded DNA (dsDNA).

In another preferred embodiment, the cytosine deaminase includes but is not limited to an enzyme selected from the group consisting of: AID (e.g., human AID), APOBEC3G (e.g., human APOBEC3G), APOBEC1, APOBECA3A, CDA1 (e.g. lamprey CDA1).

In another preferred embodiment, the weakening is a significant weakening, for example, the weakening reduces the binding ability of cytosine deaminase to DNA (preferably ssDNA) by 10%, 20%, 30%, 40%, 50%, 60%, 70%, 80%, 90% or more, or reduced by 100%.

In another preferred embodiment, the cytosine deaminase is APOBEC1; preferably, the modification is to modify the amino acid at position 126 of the enzyme; more preferably, the modification is to alter R126 of the enzyme to E.

In another preferred embodiment, the modification further includes: modification of the amino acid at position 132 of the APOBEC1 enzyme; preferably, the modification is to alter the amino acid at position 132 to E.

In another preferred embodiment, the modification further includes: modification of the amino acid at position 90 of the APOBEC1 enzyme; preferably, the modification is to alter the amino acid at position 90 to Y.

In another preferred embodiment, the modification further includes: modification of the amino acid at position 90 of the APOBEC1 enzyme; preferably, the modification is to alter the amino acid at position 90 to F.

In another preferred embodiment, the modification further includes: modification of the amino acid at position 90 and amino acid 126 of APOBEC1 enzyme, W90Y and R126E.

In another preferred embodiment, the modification further includes: modification of the amino acid at position 90 and amino acid 126 of APOBEC1 enzyme, W90F and R126E.

In another preferred embodiment, the cytosine deaminase is APOBECA3A, the modification is to modify the amino acid at position 130 of the enzyme; more preferably, the modification is to alter Y130 of the enzyme to F.

Another aspect of the present invention provides a mutant of cytosine deaminase, wherein its DNA binding region is modified to weaken its binding to DNA, such as single-stranded DNA.

In a preferred embodiment, the cytosine deaminase includes but is not limited to an enzyme selected from the group consisting of: AID, APOBEC3G, APOBEC1, APOBECA3A, CDA1.

In another preferred embodiment, the enzyme is APOBEC1; preferably, the modification occurs at or near position 126 of the domain; more preferably, the modification is to alter R at position 126 to E.

In another preferred embodiment, the enzyme is APOBEC1; preferably, the modification occurs at or near position 132 of the domain; more preferably, the modification is to alter R at position 132 to E.

In another preferred embodiment, the modification further occurs at amino acid at position 90 of the APOBEC1 enzyme; preferably, the modification is to alter the amino acid at position 90 to Y.

In another preferred embodiment, the enzyme is APOBECA3A, the modification occurs at or near the amino acid at position 130 of the enzyme; more preferably, the modification is to alter Y at position 130 to F.

In another aspect of the present invention, an isolated polynucleotide encoding the mutant is provided.

In another aspect of the present invention, a vector is provided, which contains the polynucleotide.

In another aspect of the present invention, a genetically engineered host cell is provided, which contains the vector or has the polynucleotide integrated into the genome.

In another aspect of the present invention, a single-base editor is provided, which includes the mutant of the cytosine deaminase; preferably, the editor is a BE3 single-base editor.

Another aspect of the present invention provides a method for producing the cytosine deaminase mutant, comprising the steps of: (1) culturing the host cell to obtain a culture; and (2) isolating the cytosine deaminase mutant from the culture.

Another aspect of the present invention provides the use of the cytosine deaminase mutant in gene editing based on a single-base editor system to reduce the off-target effect of the gene editor.

In a preferred embodiment, the use of the cytosine deaminase mutant may be a non-therapeutic use.

Another aspect of the present invention provides a method for screening substances useful for reducing off-target effect of a single-base editor, including: (1) treating a system with candidate substance(s), the system containing interaction (binding) between a cytosine deaminase or its DNA binding domain and DNA (such as ssDNA); and (2) detecting the interaction between the cytosine deaminase DNA binding domain and DNA in the system; wherein, if the candidate substance inhibits, blocks or down-regulates the interaction between the cytosine deaminase or its DNA binding domain and DNA, the candidate substance is useful for reducing the off-target effect of a gene editor.

In a preferred embodiment, the candidate substance includes (but is not limited to): small molecule compounds, binding molecules (such as antibodies or ligands) designed for cytosine deaminase or its DNA binding domain or a encoding nucleic acid thereof, blocking molecules (such as blockers based on amino acid modifications), interfering molecules, gene editing reagents, nucleic acid inhibitors; and/or

In another preferred embodiment, the system includes (but is not limited to): cell system (such as cells expressing cytosine deaminase or its DNA binding domain and containing DNA (such as ssDNA)) (or cell culture system), subcellular system, solution system, tissue system, organ system or animal system.

Another aspect of the present invention provides a method (GOTI) for analyzing the targeted effect of a single-base gene editing tool, the method includes the steps of: (1) obtaining a n-cell stage embryo, gene editing 1 to n-1 cells thereof; leaving at least one or a few cells unedited; wherein n is a positive integer from 2 to 10; (2) observing the occurrence and development of gene editing in the downstream development stage of the embryo.

In a preferred embodiment, in step (1), n is a positive integer of 2-8, 2-6 or 2-4; preferably, n is 2.

In another preferred embodiment, the method is an *in vitro* cultivation method or an *in vivo* cultivation method.

In another preferred embodiment, during the cleavage stage of the embryo, the edited blastomere and the unedited blastomere of the same embryo can be separated and transplanted into recipients (such as mice) to develop separate adults.

In another preferred embodiment, in step (2), the downstream development stage of the embryo is from gastrulation stage of the embryo to prenatal stage, or from embryo implantation into a uterus to prenatal stage *in vivo.*

In another preferred embodiment, the embryo is a mouse embryo, and the downstream development stage of the embryo is the 8th to 20th day of embryonic development (E8-E20 stage), preferably is the 9.5th to 18.5th day of embryonic development (E9.5-E18.5 stage), more preferably is the 12th to 16th day of embryonic development (E11-E16 stage, such as E14.5).

In another preferred embodiment, the gene editing include (but are not limited to): CRISPR-mediated gene editing, BaseEditor (base editor)-mediated gene editing, Cre/loxP-mediated gene editing, adenine base editor-mediated gene editing.

In another preferred embodiment, the CRISPR-mediated gene editing includes (but not limited to): CRISPR/Cas9-mediated gene editing, CRISPR/Cas9n-mediated gene editing, CRISPR/Cas13 (such as CRISPR/Cas13a, CRISPR/Cas13d)-mediated gene editing, CRISPR/CasRx-mediated gene editing.

In another preferred embodiment, the BaseEditor includes: BE1, BE2, BE3, BE4, BE4-Max.

In another preferred embodiment, the adenine base editor includes: ABE7.10, ABE6.3, ABE7.8, ABE7.9, Prime Editing.

In a preferred embodiment, step (1) includes: introducing a coding sequence of an enzyme (such as Cas mRNA, Crc mRNA) for cutting a nucleic acid (such as DNA) target site together with a corresponding guide sequence (such as sgRNA) into one of the cells, and performing gene editing.

In another preferred embodiment, the enzyme for cutting a nucleic acid (such as DNA) target site is selected from but not limited to the group consisting of: Cas9, Cas9n, Cas13a, CasRx, BE1, BE2, BE3, BE4, BE4 -Max, ABE7.10, ABE 6.3, ABE 7.8, ABE 7.9, Prime Editing.

In another preferred embodiment, in step (1), a detectable marker is used to label the gene editing, and the gene editing is performed on 1 to n-1 of the cells and labeled by the detectable marker.

In another preferred embodiment, the detectable marker includes but is not limited to: a dye marker, a fluorescent signal molecule, a reporter gene; more preferably, the detectable marker is (but not limited to) tdTomato, EGFP, mCherry, GFP, dsred.

In another preferred embodiment, in step (2), observing the occurrence and development of gene editing includes:
sorting cells that have undergone gene editing (such as tdTomato positive cells) and cells that have not undergone gene editing (such as tdTomato negative cells);

In another preferred embodiment, during the cleavage stage of the embryo, the edited blastomcrc and the unedited blastomere of the same embryo can be separated and transplanted into recipients (such as mice) to develop separate adults, wherein flow cytometry is not used for sorting.
analyzing by sequencing (such as WGS analysis);
analyzing through single-nucleotide variants (SNVs) analysis tools and/or indel analysis tools;
comparing edited cells with unedited cells to identify on-target effects or off-target effects, including detection of SNVs and indels.

In another preferred embodiment, the SNV analysis tool includes but is not limited to: Mutect2, Lofreq and Strelka or a combination thereof; or, the indel analysis tool includes but is not limited to: Mutect2, Scalpel, Strelka or a combination thereof.

In another preferred embodiment, flow cytometry is used to sort cells that have undergone gene editing (such as tdTomato positive cells) and cells that have not undergone gene editing (such as tdTomato negative cells).

In another preferred embodiment, the method (GOTI) for analyzing the on-target effect of a single-base gene editing tool may be a non-therapeutic method.

In another preferred embodiment, the method (GOTI) for analyzing the on-target effect of a single-base gene editing tool may be an *in vitro* method.

In another preferred embodiment, the embryo is derived from a mammal, including but not limited to a non-human mammal, such as a mouse, a rabbit, a sheep, a cow, a monkey and the like.

Other aspects of the disclosure will be apparent to those skilled in the art based on the disclosure herein.

### BRIEF DESCRIPTION OF DRAWINGS

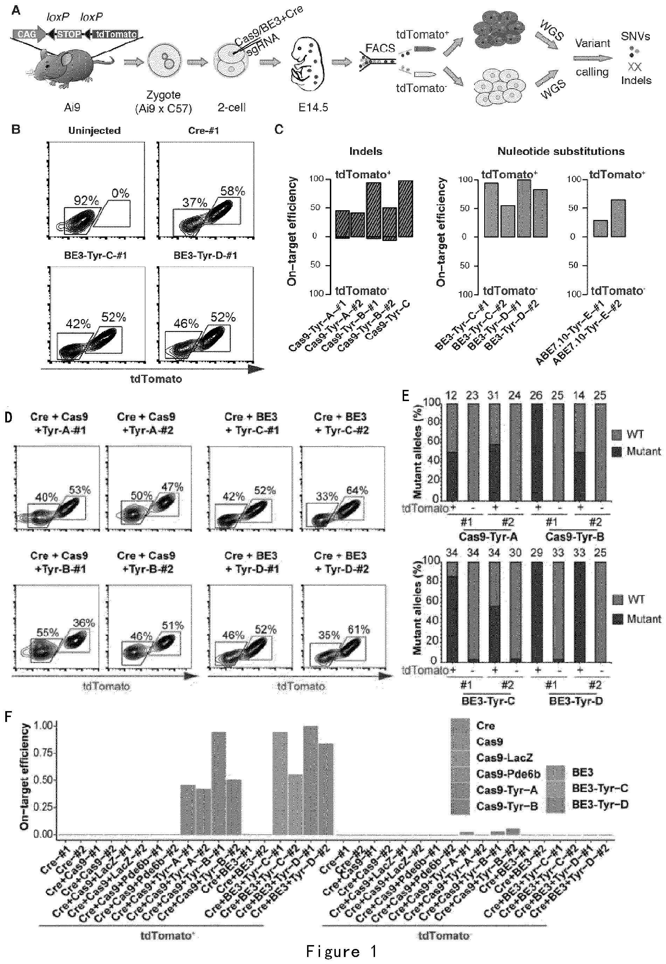

Fig. 1. CRISPR-Cas9-, BE3-, or ABE7.10-mediated gene editing in one blastomere of two-cell embryos. (A) Experimental design: a mixture of Cre, Cas9/BE3 and sgRNA was injected into one blastomere in 2-cell embryos, which were derived from the mating of male Ai9 mice with a wild-type female mice. Cre is expected to produce a chimeric embryo, half of which are labeled by tdTomato (red). TdTomato+ cells and tdTomato- cells of E14.5 chimeric embryos were separated by flow cytometry and used for whole-genome sequencing. By comparing tdTomato+ cells with tdTomato- cells, three different calling algorithms are used to identify off-target SNVs and indels (SNV: Mutect2, Lofreq and Strelka, and indel: Mutect2, Scalpel and Strelka). SNVs and indels are marked as colored dots and crosses. (B) FACS analysis in designated embryos. (C) On-target efficiency for tdTomato+ and tdTomato- cells on the basis of WGS. On-target efficiencies of Cas9, BE3, and ABE7.10 in tdTomato+ cells were 66% ± 12% insertion or deletion (SEM, n = 5), 83% ± 10% (SEM, n = 4), and 47% ± 18% (SEM, n = 2) single-base editing, respectively. (D) Flow cytometry analysis of E14.5 embryos treated with Cas9-Tyr-A, Cas9-Tyr-B, BE3-Tyr-C, and BE3-Tyr-D; flow cytometry analysis of uninjected embryos is shown in Figure S1b. (E) On-target efficiency based on whole-genome sequencing of tdTomato+ cells (left) and tdTomato- cells (right); cells treated in the same way are indicated by the same color. (F) TA clone sequencing On-target analysis of E14.5 embryos treated with Cas9-Tyr-A, Cas9-Tyr-B, BE3-Tyr-C, and BE3-Tyr-D: the number in each column represents the total number of analyzed clones.

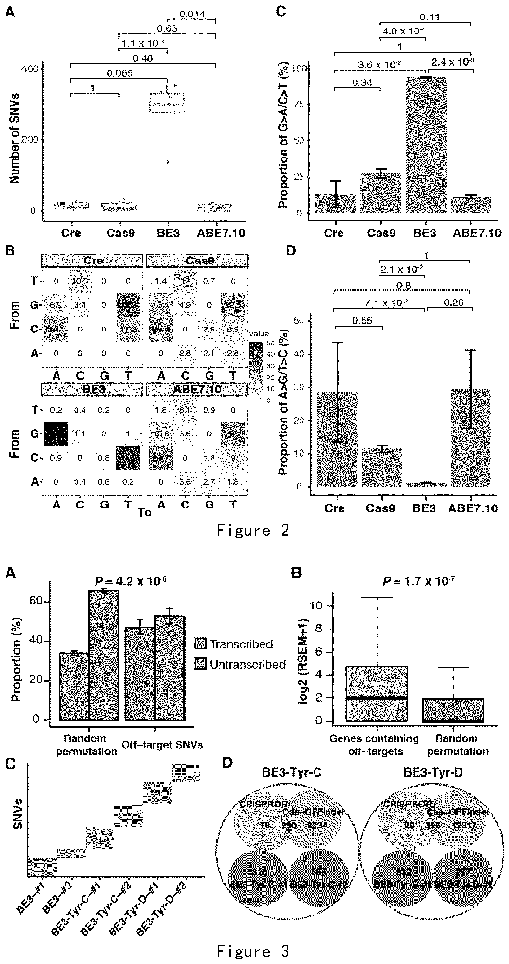

Figure 2. A large number of off-target SNVs generated in BE3-treated mouse embryos. (A) Comparison of the total number of detected off-target SNVs. The number of SNVs for Cre-, Cas9-, BE3-, and ABE7.10-treated embryos were 14 ± 12 (SEM, n = 2), 12 ± 4 (SEM, n = 11), 283 ± 32 (SEM, n = 6), and 10 ± 5 (SEM, n = 4) SNVs, respectively. (B) Distribution of mutation types. The number in each cell indicates the proportion of a certain type of mutation among all mutations. (C) Proportion of C>T and G>A mutations for Cre, Cas9, BE3, and T>C and A>G mutations for ABE7.10 groups. (D) Proportion of A>G to T>C mutations for Cre, Cas9, BE3, and ABE7.10. Two Cre, 11 Cas9, 6 BE3, and 4 ABE7.10 samples were analyzed. In (A) and (B), the P values were calculated by two-sided Wilcoxon.
Fig. 3. Characteristics of BE3-induced off-target SNVs. (A) Off-target SNVs are enriched in the transcribed regions of the genome compared with random permutation. (B) Genes containing off-target SNVs were significantly more highly expressed than random simulated genes in four-cell embryos. (C) SNVs identified from each embryo were nonoverlapping. (D) Overlap among SNVs detected by GOTI with predicted off-targets by Cas-OFFinder and CRISPOR. In (A) and (B), the P values were calculated by two-sided Wilcoxon.

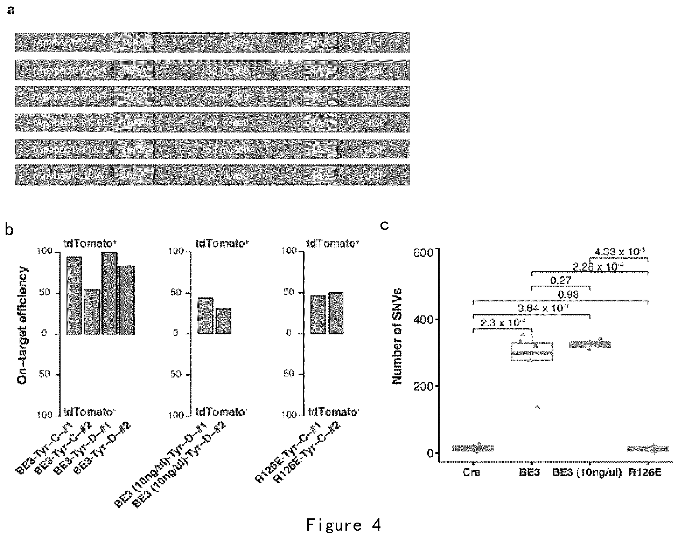

Figure 4. BE2 system constructed based on BE3 off-target evaluation. (a) Plasmid. (b) On-target efficiency of R126E mutated BE3 from WGS data. (c) Comparison of the total number of detected off-target SNVs.

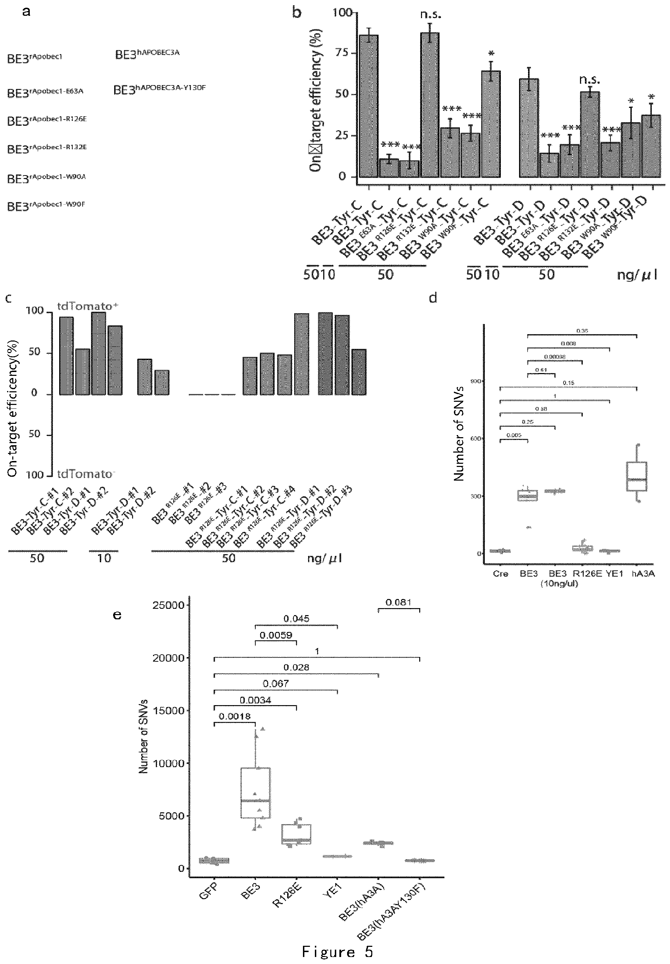

Figure 5. Apobec1 point mutation can eliminate BE3 off-target for DNA and RNA. (a) APOBEC1 in the BE3 system; (b) the correlation between the amount of BE3 (BE3 concentration by microinjection) and the on-target efficiency; (c) on-target efficiency identified by sequencing; (d) comparison of the off-target effects of different mutants. It shows that DNA off-target of BE3^{R121E}, BE3^{R126E+W90Y} is significantly reduced; (e) the correlation analysis between mutants and off-target effects.

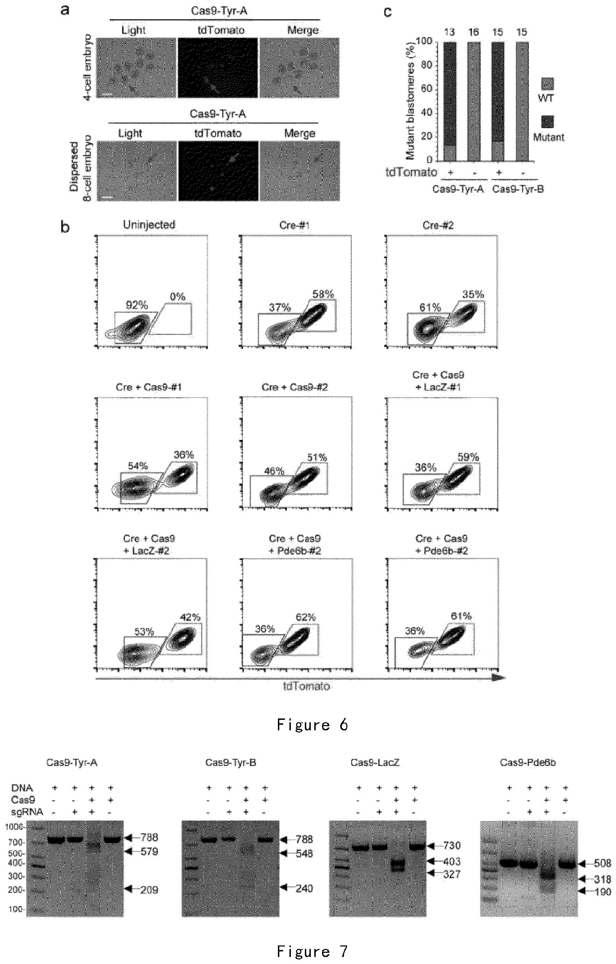

Figure 6. Flow cytometric analysis of E14.5 embryos treated with different mixes. (a) Representative image of Cas9-Tyr-A gene targeting embryos. Upper penal: four-cell embryo, lower penal: a scattered 8-cell embryo. The red arrow indicates tdTomato+blastomere. Scale bar: 100µm. (b) Left to right, top to bottom: no injection, Cre-#1, Cre-#2, Cre+Cas9-#1, Cre+Cas9-#2, Cre+Cas9+LacZ-#1, Cre+Cas9+LacZ-#2, Cre+Cas9+Pde6b-#1, and Cre+Cas9+Pde6b-#2. (c) The genotype of 8-cell embryos targeting Tyr gene. Single tdTomato+ and tdTomato- cells were isolated from four Cas9-Tyr-A and four Cas9-Tyr-B gene targeted blastocysts. Number: Total number of blastocysts analyzed. WT: wild-type allele. Mutant: Tyr allele mutation.
Figure 7. The cleavage efficiency of sgRNAs was determined by DNA *in vitro* cleavage method. Agarose gel electrophoresis shows (left to right) Cas9-Tyr-A, Cas9-Tyr-B, Cas9-LacZ and Cas9-Pde6b, respectively. PCR amplification was performed on the genomic regions or structures flanking both sides of the sgRNA target site of each gene, and the PCR products were incubated with Cas9 ribonucleoprotein and sgRNA for 3 hours.

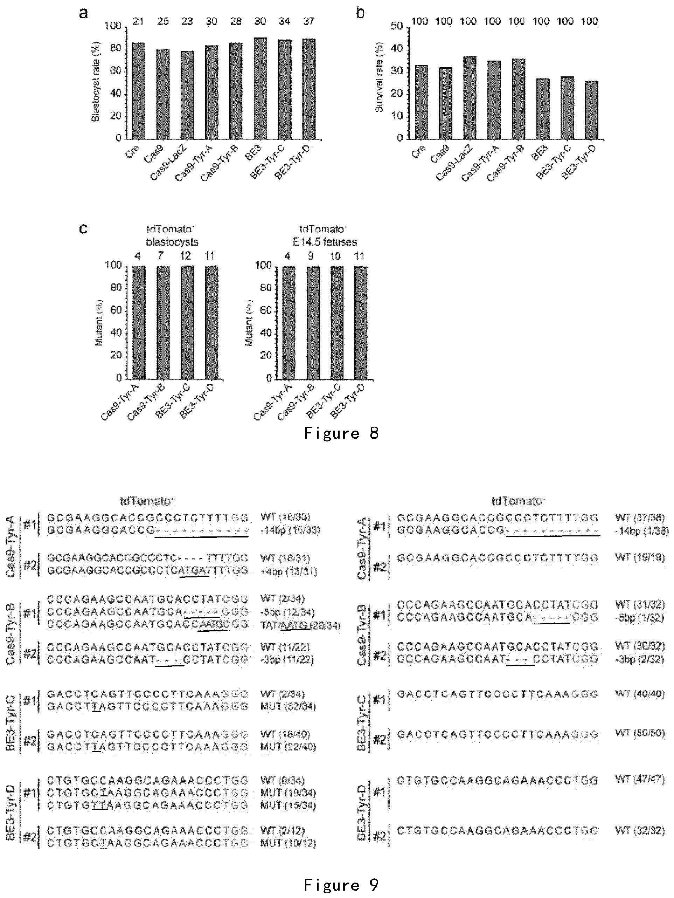

Figure 8. Development and genotype of chimeric embryos treated with CRISPR/Cas9 and BE3. (a) The percentage of tdTomato+ blastocysts after injection of different mixes. Number: Total number of blastocysts or embryos. (b) The survival rate of E14.5 embryos after injection of different mixes. Number: the total count number of embryos or the total number of transfers. (c) The percentage of tdTomato+ blastocysts and the percentage of Tyr mutations in E14.5 embryos. Number: Total number of blastocysts or embryos analyzed.
Figure 9. On-target sequence obtained by whole genome sequencing of tdTomato+ and tdTomato- cells. The WGS results of WT and mutant sequences are displayed as WT and MUT. The number before the slash indicates the WT or mutant sequence, and the total number is shown after the slash. The mutant sequence is underlined, and the last three positions TGG, CGG, and GGG are PAM.

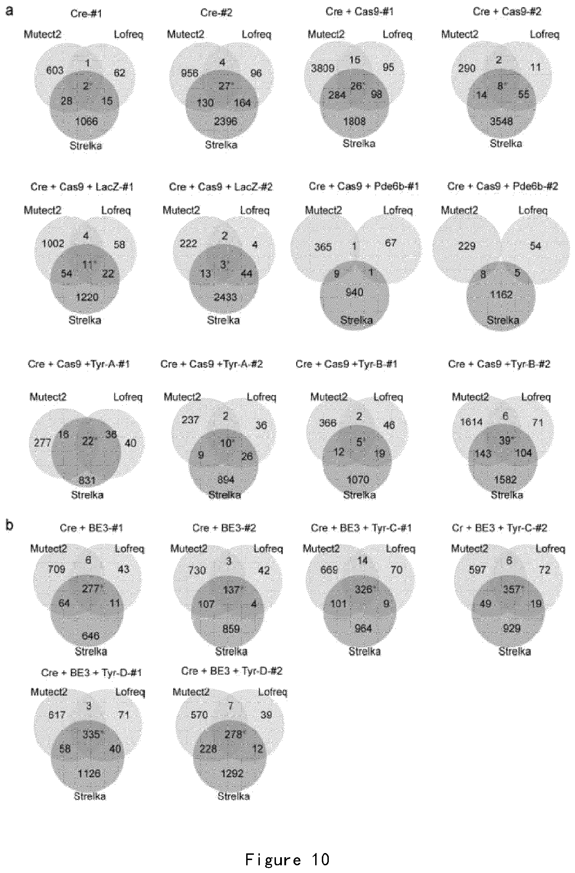

Figure 10. The Venn diagram of SNVs detected in the WGS data in each embryo using the designated software tool. (a) SNVs detected in samples processed with Cre or CRISPR/Cas9. (b) SNVs identified in BE3 treated embryos. Repeated SNVs with an allele frequency of less than 10% were not included in the subsequent analysis.

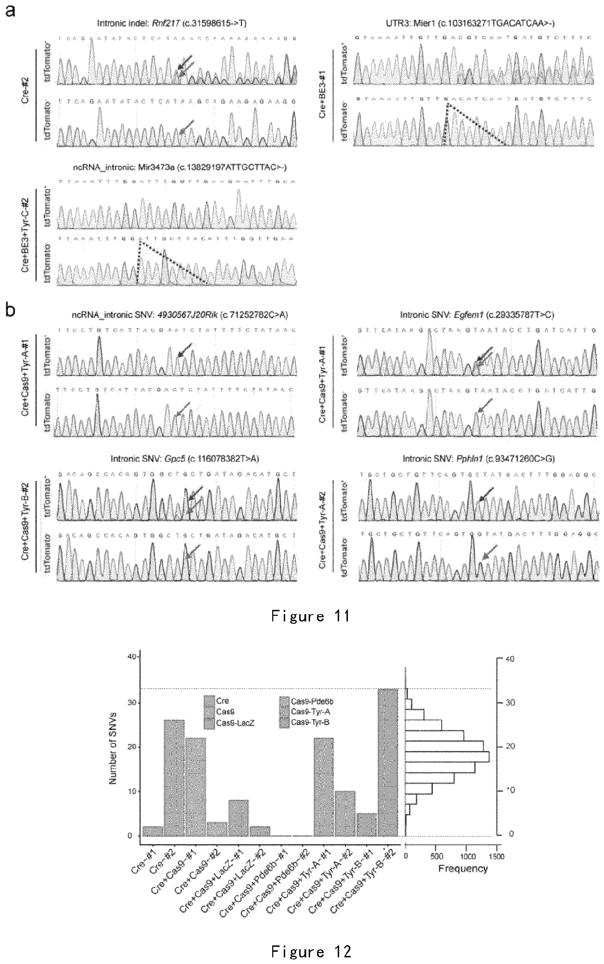

Figure 11. Representative Sanger sequencing peak map showing the detection of mutations in Cre or CRISPR/Cas9-treated embryos by whole-genome sequencing, (a) Samples were amplified by PCR and sequenced by Sanger sequencing. Green arrow: wild type; red arrow: inserted nucleotide. Red dotted line, missed nucleotides. (b) The SNVs of the samples were verified by Sanger sequencing. Green arrow: wild-type nucleotide; red arrow: mutated nucleotide. The primers are shown in Table S16.
Figure 12. The number of SNVs detected from WGS data in embryos treated with Cre and CR1SPR/Cas9. Embryos of the same group are represented by the same color. Right: the bar graph simulation-the distribution of the number of spontaneous mutations.

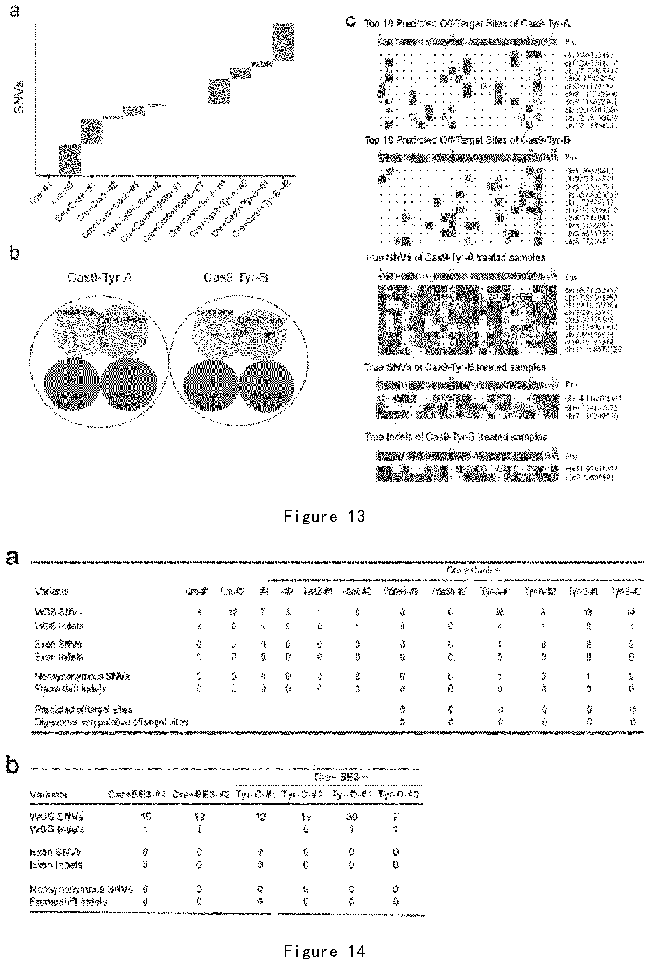

Figure 13. Off-target SNVs and indels identified from embryos treated with Cre and CRISPR/Cas9. (a) The SNVs identified in each embryo injected with Cre or CRISPR/Cas9 are mutually exclusive, (b) The overlap of SNVs detected from CRISPR/cas9-treated embryos with the off-target sites predicted by Cas-OFFinder and CRISPOR. (c) The top 10 predicted Cas9-Tyr-A and Cas9-Tyr-B Off-target sequence alignments, and the Cas9-Tyr-A and Cas9-Tyr-B mutations detected from the WGS data.
Figure 14. By comparing tdTomato- and tdTomato+ cells from the same embryo, the variation that was called back from WGS data is summarized, (a) Call the opposite variables from the samples processed by Cre- and CRISPR/Cas9. (b) Call the opposite results from the samples processed by BE3.

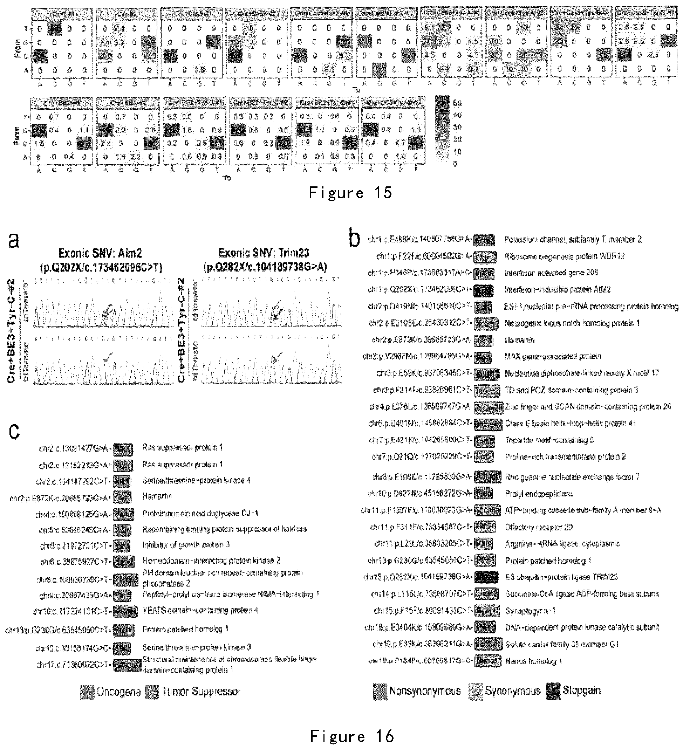

Figure 15. The type of mutation in each embryo identified in this study. The number of each compartment indicates the proportion of a certain mutation type, and the darker the color, the higher the proportion of the mutation type.
Figure 16. Comparison of the presence of identified off-target peaks among four Cistrome data sets. The number at the top of each bar represents the GEO accession of the applied data set. P value is calculated by Wilcoxon rank sum test.

### DETAILED DESCRIPTION

Genome editing is expected to correct disease-causing mutations. However, due to single nucleotide polymorphisms between different individuals, it is difficult to determine the off-target effects of gene editing. In order to study such off-target effects, the inventors developed a method for whole-genome off-target analysis by two- or multi-cell (preferably two-cell) embryo injection, named GOTI. The method of the present invention is suitable for tracking analysis detection of on-target effect/efficiency upon CRISPR-mediated gene editing, BaseEditor-mediated gene editing, Cre/loxP-mediated gene editing, adenine base editor-mediated gene editing.

The present invention provides a method (GOTI) for analyzing the targeted effect of a single-base gene editing tool, the method includes the steps of: (1) obtaining a n-cell stage embryo, gene editing 1 to n-1 cells thereof; where n is a positive integer from 2 to 10; (2) observing the occurrence and development of gene editing in the downstream development stages of the embryo. In some preferred embodiments, n is a positive integer of 2-8, 2-6 or 2-4. In a preferred embodiment, n is preferably 2.

The method of the present invention is suitable for embryo culture *in vitro,* for example, embryo culture in a test tube or other embryo culture container. The method of the present invention is also suitable for embryo cultivation in vivo, for example: performing the method of the present invention *in vitro,* transplanting the developed cells into the body, (for example transplanting into the fallopian tube of an animal, then the embryo can swim by itself into the uterus; or transplanting into the uterus of an animal).

The method of the present invention is suitable for embryo culture *in vitro,* for example, embryo culture in a test tube or other embryo culture container.

The method of the present invention is suitable for embryo culture in vitro, embryo culture in an embryo culture container, to establish an embryonic stem cell line.

The method of the present invention is suitable for embryo culture in vitro, embryo culture in an embryo culture container, to establish an embryonic stem cell line from the edited blastomere and the unedited blastomere, respectively.

The method of the present invention is suitable for the same embryo to separate the edited blastomere and the unedited blastomere and form two embryos which are respectively transplanted into recipients (different mice) or used to establish embryonic stem cell lines in vitro.

The method of the present invention is suitable for the same embryo to separate the edited blastomere and the unedited blastomere and form two embryos which are transplanted into the same recipient (one mouse) or used to establish embryonic stem cell lines in vitro.

The method of the present invention is also suitable for embryo cultivation in vivo, for example: performing the method of the present invention *in vitro,* transplanting the developed cells into the body, (for example transplanting into the fallopian tube of an animal, then the embryo can swim by itself into the uterus; or transplanting into the uterus of an animal).

In a preferred embodiment, the downstream development stages of the embryo are from gastrulation stage of the embryo to prenatal stage, or from embryo implantation into a uterus to prenatal stage *in vivo.* The inventor found that it is ideal to sort cells and determine the effect of gene editing at the "appropriate time" of embryonic development. Generally, the "appropriate time" is the stage where the embryo grows to a stage suitable for being broken down into single cells by enzymes. For example, n-cell stage embryo is a mouse embryo, and the downstream development stage of the embryo is the 8th to 20th day of embryonic development (E8-E20 stage), preferably is the 9.5th to 18.5th day of embryonic development (E9.5-E18.5 stage), more preferably is the 12th to 16th day of embryonic development (E11-E16 stage, such as E14.5).

The method of the present invention is applicable to a variety of single-base gene editing methods. The method of the present invention can be adopted in gene editing involving various enzyme(s) that cuts DNA target sites. The enzymes that cut the DNA target site can be a variety of enzymes involved in this process familiar to those skilled in the art, such as but not limited to the group consisting of: Cas9, Cas9n, Cas13a, CasRx, BE1, BE2, BE3, BE4, ABE7.10, ABE 6.3, ABE 7.8, ABE7.9, Prime Editing.

In the GOTI method, detectable markers can be used to label the gene editing. The detectable markers include, but are not limited to: dye markers, fluorescent signal molecules, and reporter genes. In the embodiment of the present invention, tdTomato is used, which is a preferred solution. Other markers can also be applied to the present invention.

As a preferred emobidment, observing the occurrence and development of gene editing includes: sorting cells that have undergone gene editing (such as tdTomato positive cells) and cells that have not undergone gene editing (such as tdTomato negative cells); analyzing by sequencing (such as WGS analysis); analyzing through SNV analysis tools and/or indel analysis tools; comparing edited cells with unedited cells to identify off-target SNVs and indels. It should be understood that the sequencing tools and analysis tools are not limited to those listed above and in the embodiments of the present invention. Other sequencing tools and analysis tools may also be applied to the present invention. Various methods known in the art can be used for cell sorting, such as but not limited to magnetic bead method, flow cytometry and the like.

In the present invention, the term "animal" refers to a mammal, including a human, a non-human primate (a monkey, an orangutan), a domestic animal and an agricultural animal (for example, a pig, a sheep, a cattle), a rat (a mouse), and a rodent (e.g., a mouse, a rat, a rabbit), etc. The animal is an animal that does not include a human; in limited or special circumstances, the animal can also be a human, but this is only suitable for an application that does not involve "commercial applications of human embryos".

In a specific embodiment of the present invention, the comparison of the whole genome sequence of the progeny cells of edited and unedited blastomeres at E14.5 showed that in CRISPR-Cas9 or adenine single-base edited embryos, single-nucleotide vibration (SNV) off-target is rare, with a frequency close to the spontaneous mutation rate. In contrast, cytosine single-base editing induces more than 20-fold off-target single-nucleotide vibrations.

Before clinical application, mammalian cells are required to have no genome-wide off-target. However, due to the nucleotide polymorphisms in individuals, it is difficult to determine the extent of off-target effects. The GOTI (genome-wide off-target analysis by two-cell embryo injection) method developed by the present invention changes this current situation, which detects off-target mutations without interfering with SNPs, and can accurately and effectively analyze genome on-target effects.

The present inventors further studied the causes of off-target effects (such as single-nucleotide off-target mutations) in single-base editing. Upon observing that the single-base editing tool BE3 will cause a large number of single nucleotide off-target variants (SNVs), the inventors conducted a lot of research work and finally determined that these off-target mutations were caused by the overexpression of APOBEC1 and its binding with DNA (such as ssDNA). In a specific embodiment, the present invention discloses a solution to solve the off-target effect induced by BE3 by adding mutation(s) on APOBEC1, such as R126E, R132E, W90F, W90Y and W90F/R126E, W90Y/R126E mutation(s).

As mentioned above, the present invention has determined a useful method for reducing the off-target effect of single-base editors, including: modifying the cytosine deaminase in the single base editor system to weaken its binding to DNA (such as ssDNA). Preferably, the modification is the modification of the DNA binding region of cytosine deaminase; more preferably, the DNA binding region is a domain that binds to DNA. The single-base editor is, for example, the BE3 gene editor.

A variety of modification methods for cytosine deaminase can be used herein, as long as the weakening effect can be realized. As an alternative, the modification may includes: gene mutation, targeted blocking (such as blocking by binding proteins or antibodies, or blocking by competitive binding molecules), interference, etc.

A variety of cytosine deaminase that can be applied to the single-base editor system or enzymes having the same function can be modified by the method of the present invention to reduce the off-target effect of the single-base editor system. For example, the cytosine deaminase includes but is not limited to an enzyme selected from the group consisting of: AID (e.g., human AID), APOBEC3G (e.g., human APOBEC3G), APOBEC1, CDA1 (e.g. lamprey CDA1).

In the present invention, the term "weaken" or "weakening" means that the interaction (binding) ability of a cytosine deaminase with DNA is down-regulated or eliminated. For example, the weakening reduces the binding ability of cytosine deaminase to DNA by 10%, 20%, 30%, 40%, 50%, 60%, 70%, 80%, 90% or more, or 100%.

As a preferred embodiment of the present invention, a specific cytosine deaminase APOBEC1 (see SEQ ID NO: 1 for the wild-type sequence, and SEQ ID NO: 4 for a mutant thereof) is provided. After modification of the enzyme's DNA binding region, the editing results of the single-base editor system involving the enzyme have changed substantially, with the off-target effect significantly reduced. Preferably, such modification is to modify the amino acid at position 126 of the enzyme, more preferably, the modification is to mutate the R at position 126 to E.

In a more preferred embodiment, the modification of APOBEC1 further occurs at amino acid at position 90 of the APOBEC1 enzyme; preferably, the modification is to alter the amino acid at position 90 to Y.

In a more preferred embodiment, the modification of APOBEC1 further occurs at the 90th amino acid of the APOBEC1 enzyme; preferably, the modification is to alter the amino acid at position 90 to Y.

As another preferred emobodiment of the present invention, a specific cytosine deaminase APOBECA3A (SEQ ID NO: 37) is provided. The modification of APOBECA3A occurs at or near the 130th amino acid of the enzyme. Preferably, the modification is to alter its (SEQ ID NO: 37) Y at position 130 to F.

Based on the inventor's discovery, further provided is a method for screening substances useful for reducing off-target effect of BE3 gene editor, including: (1) treating a system with candidate substance(s), the system containing interaction (binding) between a cytosine deaminase or its DNA binding domain and DNA; and (2) detecting the interaction between the cytosine deaminase DNA binding domain and DNA in the system; wherein, if the candidate substance inhibits, blocks or down-regulates the interaction between the cytosine deaminase or its DNA binding domain and DNA, the candidate substance is useful for reducing the off-target effect of BE3 gene editor.

In a preferred embodiment of the present invention, in order to observe changes in interaction (binding) between cytosine deaminase or its DNA binding domain and DNA during the screening, a control group can also be set. A control may be a system containing interaction (binding) between a cytosine deaminase or its DNA binding domain and DNA without adding the candidate substance.

In preferable embodiments, the method further includes: performing a cell experiment and/or animal experiment on the obtained potential substances to further select and determine a substance that is really useful for regulating the interaction (binding) between the cytosine deaminase or its DNA binding domain and DNA.

The disclosure is further illustrated by the specific examples described below. It should be understood that these examples are merely illustrative, and do not limit the scope of the present disclosure. The experimental methods without specifying the specific conditions in the following examples generally used the conventional conditions, such as those described in J. Sambrook, Molecular Cloning: A Laboratory Manual (3rd ed. Science Press, 2002) or followed the manufacturer's recommendation.

### Materials and methods

### 1. Experimental design including GOTI method

The mixture of Crc, Cas9/BE3/ABE7.10 mRNA and sgRNA were injected into one blastomcrc of two-cell embryos derived from wild-type female mice X Ai9 male mice. The addition of Cre produces chimeric embryos in which the injected cells are marked with tdTomato (red). A positive tdTomato indicates that editing has occurred, and a negative tdTomato indicates unedited cells.TdTomato positive cells and tdTomato negative cells were separated from chimeric embryos by FACS at E14.5 and used for WGS analysis respectively. Off-target SNVs and indels were identified by comparing tdTomato+ cells and tdTomato- cells using three algorithms (Mutect2, Lofreq and Strelka for SNV analysis, and Mutect2, Scalpel and Strelka for indel analysis).SNVs and indels are represented as colored dots and crosses in Figure 1A.The Cre protein sequence is shown in SEQ ID NO: 2, and the Cas9 protein sequence is shown in SEQ ID NO: 3.

### 2. Animals and care

Female C57BL/6 mice (4 weeks old) and heterozygous Ai9 (B6.Cg-Gt(ROSA)26Sortm9(CAG-td-Tomato)Hze/J; JAX strain 007909) male mice were used for embryo collection.ICR female mice are used as recipients.The treatment and care of animals conform to the guidelines of the Biomedical Research Ethics Committee of the Shanghai Institutes for Biological Sciences, Chinese Academy of Sciences.

### 3. Cas9 mRNA, BE3 mRNA, ABE7.10 mRNA, Cre mRNA and sgRNA

The Cas9 protein coding region was amplified from the px260 plasmid using primers Cas9F and R.Purify the T7-Cas9 PCR product, and use mMESSAGE mMACHINE T7 ULTRA to transcribe mRNA.T7-sgRNA PCR was amplified from the px330 plasmid and transcribed into RNA in vitro using MEGA Shortcript T7 kit (Life Technologies).The T7 promoter was added to the Cre template by PCR amplification, and the T7-Cre PCR product was purified, and it was transcribed into mRNA in vitro using the mMESSAGE mMACHINE T7 ULTRA kit (Life Technologies). Use MEGA clear kit (Life Technologies) to purify Cas9 mRNA, Cre mRNA and sgRNA, and elute in RNase-free water.

| sgRNA sequence (from top to bottom: SEQ ID NO: 5-11) | |
|---|---|
| Locus Tyr-A (*22*) | Sequence (5'-3') GCGAAGGCACCGCCCTCTTTTGG |
| Tyr-B (*22*) | CCAGAAGCCAATGCACCTATCGG |
| LacZ (*23*) | TGCGAATACGCCCACGCGATGGG |
| Pde6b (*24*) | CCAACCTAAGTAGCAGAAAGTGG |
| Tyr-C (*11*) Tyr-D | GACCTCAGTTCCCCTTCAAAGGG CTGTGCCAAGGCAGAAACCCTGG |
| Tyr-E | CCATAACAGAGACTCTTACATGG |

| Primer sequence (from top to bottom: SEQ ID NO: 12-26) | |
|---|---|
| Name | Sequence (5'-3') |
| Cre IVT F | TAATACGACTCACTATAGGGAGACAGATCACCTTTCCTAT CAACC |
| Cre IVT R | TCGGTATTTCCAGCACACTGGA |
| BE3 IVT F | TCCGCGGCCGCTAATACGACT |
| BE3 IVT R | TGGTTCTTTCCGCCTCAGAAGCC |
| Cas9 IVT F | TAATACGACTCACTATAGGGAGATTTCAGGTTGGACCG GTG |
| Cas9 IVT R | GACGTCAGCGTTCGAATTGC |
| ABF7.10 IVT F | GAGGTCTATATAAGCAGAGCTC |
| ABE7.10 IVT R | ATTAATAACTAGCGGCCGCTCCC |
| Tyr-AIVT F | TAATACGACTCACTATAGGGGCGAAGGCACCGCCCTCT TTGTTTTAGAGCTAGAAATAG |
| Tyr-B IVT F | TAATACGACTCACTATAGGGCCAGAAGCCAATGCACCT ATGTTTTAGAGCTAGAAATAG |
| Tyr-C IVT F | TAATACGACTCACTATAGGGGACCTCAGTTCCCCTTCA AAGTTTTAGAGCTAGAAAIAG |
| Tyr-D IVT F | TAATACGACTCACTATAGGGCTGTGCCAAGGCAGAAA CCCGTTTTAGAGCTAGAAATAG |
| Tyr-E IVT F | TAATACGACTCACTATAGGGCCATAACAGAGACTCTTAC AGTTAGAGCTAGAAATAG |
| LacZ-IVT F | TAATACGACTCACTATAGGGTGCGAATACGCCCACGCGA TGTTTTAGAGCTAGAAATAG |
| sgRNA IVT R | AAAAGCACCGACTCGGTGCC |

### 4. 2-Cell injection, embryo culture and embryo transfer

Superovulate C57BL/6 females (4 weeks old) mated with heterozygous Ai9 B6.Cg-Gt(ROSA)26Sortm9(CAG-td-Tomato)Hze/J; JAX strain 007909) males. 23 hours after hCG injection, fertilized eggs was taken from the fallopian tube.For 2-cell editing, a mixture of Cas9 mRNA (50ng/µl), BE3 mRNA (50ng/µl) or ABE7.10 mRNA (50ng/µl), sgRNA (50ng/µl) and Crc mRNA (2ng/µl) in a drop of HEPES-CZB medium containing 5µg/ml cytochalasin B (CB), was injected into the cytoplasm of one blastomere in a 2-cell embryo by FemtoJet micro-syringe (Eppendorf) at a constant flow, 48 hours after hCG injection. The injected embryos were cultured in KSOM medium containing amino acids at 37°C and 5% CO₂ for 2 hours, and then transplanted into the fallopian tubes of pseudopregnant ICR females.

### 5. Single cell PCR analysis

Under a dissecting microscope, 8-cell mouse embryos were digested with acid Tyrode solution to remove the zona pellucida use homemade glass capillaries, then the embryos were transfered to 0.25% trypsin and gently pipette to separate individual blastomeres.Finally, wash the blastomere in KSOM for 7 to 10 times and transfer to a PCR tube.Then 1.5 µl of lysis buffer containing 0.1% Tween 20, 0.1% Triton X-100 and 4 µg/ml proteinase K was pipetted into the tube.Each tube was centrifuged to promote mixing.The lysate was incubated at 56°C for 30 minutes, and then at 95°C for 5 minutes.The product of the lysis procedure is used as a template in nested PCR analysis. Avoid contaminating samples in all operations.

| Nest PCR Primer sequence (from top to bottom: SEQ ID NO: 27-30) | | |
|---|---|---|
| Tyr | Outer F: GTTATCCTCACACTACTTCTG | |
| | Outer R: GTAATCCTACCAAGAGTCTCA | |
| | Inner F: TCCTCACACTACTTCTGATG | |
| | Inner R: GTCTCAAGATGGAAGATCAC | |

### 6. T vector cloning and genotype testing

The PCR product was purified and ligated to pMD18-T vector and transformed into competent E. coli strain DH5α.After culturing overnight at 37°C, randomly selected clones were sequenced by the Sanger method.The genotype of mutant E14.5 embryos was determined by PCR of genomic DNA extracted from cells.ExTaq was activated at 95°C for 3 minutes; PCR was carried out for 34 cycles: 95°C for 30 seconds, 62°C for 30 seconds, 72°C for 1 minute; and finally at 72°C for 5 minutes.For embryos, after washing 6 times with KSOM, a single embryo was transferred directly to a PCR tube containing 1.5µl embryo lysis buffer (0.1% Tween 20, 0.1% Triton X-100 and 4µg/ml proteinase K) and incubated for 30 minute. At 56°C, inactivating at 95°C for 10 minutes. Nest primers were used for PCR amplification. ExTaq was activated at 95°C for 3 minutes; PCR was carried out for 34 cycles: 95°C for 30 seconds, 62°C for 30 seconds, 72°C for 1 minute; and finally at 72°C for 5 minutes.The second PCR was performed using 0.5 µg product of the first round PCR and inner primers. PCR is performed in the same reaction mixture.The PCR product was gel purified and cloned using the pMD-19t cloning kit (Takara) according to the manufacturer's instructions. Colonies was selected from each transformation and then subjected to Sanger sequencing to detect mutations.

| Primer sequence (from top to bottom: SEQ ID NO: 31-36) | |
|---|---|
| Name | Sequence (5'-3') |
| Tyr F | GTTATCCTCACACTACTTCTG |
| TyrR | GTAATCCTACCAAGAGTCTCA |
| Tyr-OF | GTCTGTGACACTCATTAACC |
| Tyr-OR | CATAGGAGGTTGCTAACAATAC |
| Tyr-IF | GTATTGCCTTCTGTGGAGTT |
| Tyr-IR | TGAACCAATCAGTCCTTGTT |

### 7. Fluorescence activated Cell Sorting (FACS)

In order to separate the cells, the shredded tissue was enzymatically hydrolyzed in 5 mL trypsin-EDTA (0.05%) solution at 37°C for 30 minutes. The digestion was stopped by adding 5 ml of DMEM medium containing 10% fetal bovine serum (FBS).Then repeatedly pipetting 30-40 times by a 1ml pipette tip to homogenize the fetal tissue.The cell suspension was centrifuged for 6 minutes (800 rpm), and the pellet was re-suspended in DMEM medium containing 10% FBS. Finally, the cell suspension was filtered through a 40-µm cell strainer, and tdtomato+/tdtomato- cells were separated by FACS. The second round was subjected to flow cytometry and fluorescence microscopy analysis and evaluation, with a sample purity >95% as qualified.

### 8. Whole genome sequencing and data analysis

According to the manufacturer's instructions, DNeasy Blood and Tissue Kit (Cat. No. 69504, Qiagen) was used to extract genomic DNA from the cells.WGS is performed by Illumina HiSeq X Ten with an average coverage rate of 50 times. BWA (v0.7.12) is used to map qualified sequencing reads to the reference genome (mm10). Then the Picard tool (v2.3.0) was used to rank and mark the duplicates of the mapped BAM file. In order to identify de novo genome-wide mutations with high confidence, three algorithms Mutect2 (v3.5), Lofreq (v2.1.2) and Strelka (v2.7.1) were used for single-nucleotide mutations (25-27) analysis.At the same time, Mutect2 (v3.5), Scalpel (v0.53) and Strelka (v2.7.1) were used to detect the whole genome sequence. The overlap of the three SNV or indel algorithms indicate the true variant. The variants were identified in the location BAM file of the tdTomato+ sample, where the tdTomato- sample is in the same embryo as the control, and only the mutant variant in the tdTomato+ sample can be identified. For example, if the WT allele is G at certain position, and tdTomato+ cells show A, and tdTomato- cells show G at the position, then mutant A will be referred to as a de novo mutation. However, if tdTomato-cells show A at the position, the mutant cannot be identified. In order to further verify that off-target SNVs are only identified in tdTomato+ samples, the inventors also used the variants in tdTomato- samples and tdTomato+ samples in the same embryo as controls, wherein only the variants were mutated in tdTomato- cells but could be identified in WT tdTomato+ cells.

WGS analysis showed that the low-level targeted editing range in tdTomato- cells in the Cas9-Tyr-A and Cas9-Tyr-B groups was 0-6.3%, which may be caused by false negative FACS sorting (known to occur in low level).Therefore, the inventors only considered that variants with an allele frequency higher than 10% are reliable in the subsequent analysis. We also marked variants that overlap with UCSC repeat regions and microsatellite sequences, or exist in dbSNP (v138) and MGP (v3) databases. All sequencing data are stored in NCBI (SRA).

In order to verify the target efficiency, we compared the BAM file with the on-target with the e-value of 0.0001.Two algorithms were used to predict the potential off-target out of on-target (Cas-OFFinder (http://www.rgenome.net/cas-offinder/) and CRISPOR (http://crispor.tefor.net) /)).

SNVs and indels were annotated using the RefSeq database by annovar (version 2016-02-01). Proto-oncogenes and tumor suppressor genes were searched from UniprotKB/Swiss-Prot database (2018-09). The inventor downloaded 5 ATAC-seq files from the CistromeDB database, wherein the biological source is embryos and passed all quality control. The five data sets retrieved include CistromeDB IDs "79877" (GSM2551659), "79976" (GSM2551677), "80493" (GSM2535470), "81049" (GSM2551664) and "81052" (GSM2551667). Based on the position in a chromosome, the off-target site is located to the peak area in each file, and then the peak areas with or without off-target are compared with each other through the two-sided Wilcoxon rank sum test.

### 9. Simulation of spontaneous mutations during embryonic development

In order to estimate the amount of spontaneous mutations from the 2-cell stage to the E14.5 stage, considering an average sequencing coverage of 40 and an allele frequency threshold of 10%, single nucleotide mutations were found in computer simulations.For each round of simulation, given the mutation rate of 1.8×10⁻¹⁰ and the size of the mouse nuclear genome (2,785,490,220bp), we considered the replication process from the 2-cell stage to the 16-cell stage. The mutation occurred after 16-cell stage will not be detected considering the allele frequency.During each replication, each cell can be mutated or not. Once a mutation occurs, the dividing cells will inherit the mutation. Then cumulative mutations and their wild-type alleles were randomly select for sequencing with a depth of 40. The selected mutations were added up as the number of spontaneous mutations in each round, and the same process was repeated 10,000 times

### 10, Digenome-seq analysis

As mentioned above (32), multiple Digenome-seq was performed, including Cas9-LacZ, Cas9-Pde6b, Cas9-Tyr-A and Cas9-Tyr-BSpecifically, TTANamp Genomic DNA Kit (Tiangen) was used to purify genomic DNA from the tail of the mouse according to the manufacturer's instructions.The sgRNA target site of each gene, including the flanking genomic region, was PCR amplified. PCR products were purified with Universal DNA Purification Kit (Tiangen) according to the manufacturer's instructions. The Cas9 protein (1 µg) and sgRNA (1 µg) were pre-incubated for 10 minutes at room temperature to form the RNP complex. The DNA (4 µg) and RNP complexes were incubated in the reaction buffer at 37°C for 3 hours. After adding RNase A (100 µg/ml) to remove sgRNA, the digested DNA was purified again with Universal DNA Purification Kit (Tiangen).

The library was sequenced (WGS) by the Illumina HiSeq X Ten sequencer at a sequencing depth of 30x to 40x. Digenome-seq2 (https://github.com/chizksh/digenome-toolkit2) was used to calculate and identify DNA cleavage sites. The *in vitro* cleavage sites were classified and identified by the R package "Biostrings" based on editing distance and listed.

### 11. Statistical Analysis

R version 3.5.1 (http://www.R-proiect.org/) was used for all statistical analysis in this disclosure. All tests are two-sided tests, and P<0.05 indicates that the difference is statistically significant.

### Example 1. Evaluation of three gene editing tools

Three commonly used gene editing tools CRISPR-Cas9, cytosine base editor 3 (BE3, rAPOBEC1-nCas9-UGI) and adenine base editor 7.10 (ABE7.10, TadA) -TadA^{∗}-nCas9) were evaluated by GOTI for off-target effects (references 6-8).

CRISPR-Cas9, BE3 or ABE7.10 together with Cre mRNA and the corresponding sgRNA were injected into one blastomere of 2-cell embryos from Ai9 (CAG-LoxP-Stop-LoxP-tdTomato) mice (References 9-10) (Figure 1A) to conduct CRISPR/Cas9 or BE3 gene editing combined with Cre mRNA editing. According to the expression of tdTomato in gene-edited cells, the edited and unedited cells in the sub-cell population were sorted by fluorescence activated cell sorting (FACS). tdTomato+ and tdTomato- were subjected to whole-genome sequencing respectively.

FACS was used to separate E14.5-day embryos and sort the cells based on the tdTomato in the cells. At such time, the whole embryo can be easily digested to obtain enough single cells (Figure IB). Figure ID shows that Flow cytometry analysis of E14.5 embryos treated with Cas9-Tyr-A, Cas9-Tyr-B, BE3-Tyr-C, and BE3-Tyr-D; flow cytometry analysis of uninjected embryos is shown in Figure 6b.TA clone sequencing On-target analysis for E14.5 embryos treated with Cas9-Tyr-A, Cas9-Tyr-B, BE3-Tyr-C, and BE3-Tyr-D is shown in Figure 1E. The targeting efficiency of tdTomato+ cells (left) and tdTomato- cells (right) based on whole genome sequencing in the present disclosure is shown in Figure 1F.

The inventors further demonstrated that edited cells treated with Cre and Cas9/BE3 systems can be effectively separated from unedited cells.During the Cre-mediated recombination process, about 50% of embryonic cells express tdTomato. This is verified by observation of 4-cell stage or 8-cell stage under a fluorescence microscope or flow cytometry analysis of E14.5-day cells, as shown in Figure 6a-b. In addition, the inventors also found that efficient targeted editing was achieved by CRTSPR/Cas9 when editing hair color gene tyrosinase by injecting any sgRNAs (Cas9-Tyr-A, Cas9-Tyr-B) into one blastomere in a 2-cell embryo. Sequencing of Tyr gene showed that 13 (Cas9-Tyr-A) and 15 (Cas9-Tyr-B) tdTomato+ cells were collected from 4 scattered 8-cell embryos, and 85% and 80% of cells contained Tyr alleles mutation. In contrast, the collected 16 (Cas9-Tyr-A) and 15 (Cas9-Tyr-B) tdTomato-cclls did not have Tyr allele mutations, as shown in Figure 6c.

Whole genome sequencing (WGS) was performed on the separated tdTomato+ and tdTomato-cells, and the tdTomato+ samples were identified by three algorithms for SNVs and indels. At the same time, the tdTomato- samples from the same embryo were used as references.

The inventors also verified the editing efficiency of this method when targeting Tyr gene. To study the embryo injection method on whole-genome sequencing, four sgRNAs were designed for CRISPR/Cas9 editing, Cas9-Tyr-A and Cas9-Tyr-B targeting to Tyr; a control sgRNAs targeting a LacZ lacking of a cleavage site in the genome of C57 mice; an sgRNA targeting Pde6b, which has a mismatch as compared with the C57 mouse genome, and is reported to capable of producing a large amount of SNVs. Through DNA cleavage experiments, the cleavage efficiency of these sgRNAs was verified *in vitro.* The results are shown in Figure 7, indicating that effective cleavage occurred.

The inventors also assayed two sgRNAs targeting Tyr gene through BE3 mediation. Three groups of embryos injected with Cre only, Cre and Cas9, Cre and BE3 were included as control groups. A mixture of CRISPR/Cas9 or BE3, Cre mRNAs and sgRNAs was injected into one blastomere, and embryo development was found to be undamaged, as shown by the normal blastocyst rate (Figure 8a) and survival rate (Figure 8b). Sanger sequencing showed that all detected blastocysts and E14.5 fetuses from Cas9-Tyr-A, Cas9-Tyr-B, BE3-Tyr-C and BE3-Tyr-D had Tyr mutations (Figure 8c). In order to verify the editing efficiency of the targeted Tyr gene, E14.5 fetuses treated with Cas9-Tyr-A or Cas9-Tyr-B were sorted by FACS, and about 400k tdTomato+ and 400k tdTomato- cells were collected for TA Clone sequencing.The experimental results show that there are 50% and 100% allelic mutations in tdTomato+ cells edited by Cas9-Tyr-A and Cas9-Tyr-B. In the second repeated experiment, the tdTomato+ cells edited with Cas9-Tyr-A and Cas9-Tyr-B had 58% and 50% allelic mutations (31 and 14 clones). In contrast, tdTomato-cells collected from E14.5-day embryos did not have Tyr allele mutations. Similarly, BE3 has a high editing efficiency, with 71% allelic mutations in BE3-Tyr-C and 100% allelic mutations in BE3-Tyr-D, while the corresponding tdTomato-cells have only about 3% Tyr mutation.These results prove that CRISPR/Cas9 and BE3 editing produces high-efficiency target editing efficiency in tdTomato+, but basically no target editing efficiency in tdTomato- cells.

In order to further explore the editing efficiency and potential whole-genome off-target effects, whole-genome sequencing were performed with an average depth of 47 (47x) on 36 samples from 18 E14.5 embryos and 9 treatments: Cre only, Cre and Cas9 , Cre and Cas9-LacZ, Cre and Cas9-Pde6b, Cre and Cas9-Tyr-A, Cre and Cas9-Tyr-B, Cre and BE3, Cre and BE3-Tyr-C, Cre and BE3-Tyr-D, of which Only Cas9-Tyr-A, Cas9-Tyr-B, BE3-Tyr-C and BE3-Tyr-D have re-editing sites in the C57 genome. On-target analysis of Cas9-Tyr-A and Cas9-Tyr-B showed that there were 56% and 72% Tyr allele mutations in tdTomato+ cells, respectively, indicating that there is a high-efficiency on-target efficiency on the Tyr gene; Similarly, BE3-Tyr-C and BE3-Tyr-D both showed high-efficiency editing in tdTomato+ cells (with an average of 75% and 92% Tyr allele mutations, respectively), as shown in Figure 9. The inventors also analyzed the on-target efficiency of other tdTomato+ embryos, and no control embryo had on-target efficiency. However, whole-genome sequencing analysis showed that Cas9-Tyr-A and Cas9-Tyr-B treated tdTomato-cells had 0-6.3% low-level targeted editing, which may be caused by false-negative flow cytometry sorting, which is already known occurs at a lower level. Therefore, in the following analysis, it is reliable to consider only variants with an allele frequency exceeding 10%.

In order to evaluate off-target effects, three different mutation calling algorithms were used in each embryo to compare tdTomato+ cells and tdTomato- cells. The inventors analyzed the genome-wide mutation throughout the whole genome. The variables defined by the three algorithms are all true variable. Only 0-4 indels were found in all 9 groups (Figure 10). This result was further verified by Sanger sequencing (Figure 11). At the same time, in Cre only embryos, an average of 14 SNVs were observed. Average SNVs of embryos treated with CRISPR/Cas9 (Cas9, Cas9-LacZ, Cas9-Pde6b, Cas9-Tyr-A and Cas9-Tyr-B) were 12.5, 5, 0, 16, 19. Compared with the "Cre-only" group, the difference was not statistically significant. In addition, it was observed that SNVs did not increase in Cas9-Pde6b edited embryos, which is consistent with many previous studies (Figure 12). All off-target SNVs detected in CRISPR/Cas9-edited embryos were confirmed by Sanger sequencing. The detection of SNVs in Cre- or CRISPR/Cas9-treated samples may be caused by spontaneous mutations during gene replication, but the amount of mutations is within the range of spontaneous mutations. In addition, the inventor's study did not show the same mutation (Figure 13a), and did not overlap with the speculated off-target sites of Cas-OFFinder, CRISPOR and Digenome-seq (Figure 13b). The inventors also found that adjacent sequences of the identified SNVs have no sequence similarity to the targeted site (Figure 13c).

In addition, by calling the opposite variables, the tdTomato- and tdTomato+ samples of each embryo were compared, it was found that the amout of SNVs was similar, indicating that CRISPR/Cas9 editing did not produce off-target effects. The SNVs observed by the inventors came from spontaneous mutations (Figure 14).

The inventors further designed 12 groups for detection: one Cre group (Cre only), six Cas9 groups with or without sgRNA (Cas9, Cas9-LacZ, Cas9-Pde6b, Cas9-Tyr-A, Cas9-Tyr- B and Cas9-Tyr-C), three BE3 groups with or without sgRNA (BE3, BE3-Tyr-C, BE3-Tyr-D) (Reference 11) and two ABE groups with or without sgRNA (ABE7.10, ABE7.10-Tyr-E).

The targeting efficiency of embryos at 8-cell and E14.5 stage was verified by Sanger sequencing. In order to further explore the editing efficiency of the target site and potential genome-wide off-target effects, 46 samples from 23 E14.5 embryos were subjected to WGS with an average depth of 47x (Table 1).

**Table 1. Summary of HiSeq X Ten Sequencing**

| **Sample** | **Group** | **Accession** | **Mapped bases (Gbp)** | **Coverage** |
|---|---|---|---|---|
| **Cre-#1** | tdTomto- | SRS2549042 | 127.72 | 45.85 |
| | tdTomato- | SRS2549043 | 130.14 | 46.72 |
| **Cre#2** | tdTomato- | SRS2549040 | 131.92 | 47.36 |
| | tdTomato- | SRS2549031 | 131.91 | 47.36 |
| **Cas9-#1** | tdTomato+ | SRS2549032 | 124.06. | 44.54 |
| | tdTomato- | SRS2549035 | 135.23 | 48.55 |
| **Cas9-#2** | tdTomato+ | SRS2G04284 | 132.48 | 47.56 |
| | tdTomato- | SRS2604285 | 132.56 | 47.59 |
| **Cas9-LacZ-#1** | tdTomato- | SRS2549038 | 128.44 | 46.11 |
| | tdTomato- | SRS2549039 | 119.22 | 42.80 |
| **Cas9-LacZ-#2** | tdTomato- | SRS2604286 | 127.49 | 45.77 |
| | tdTomato- | SRS2604287 | 140.58 | 50.47 |
| **Cas9-Pde6b-#1** | tdTomato+ | SRS3024198 | 127.20 | 45.67 |
| | tdTomato- | SRS3024199 | 122.52 | 43.98 |
| **Cas9-Pde6b-#2** | tdTomato- | SRS3024196 | 131.14 | 47.08 |
| | tdTomato- | SRS3024197 | 135.07 | 48.49 |
| **Cas9-Tyr-A-#1** | tdTomato+ | SRS2549029 | 133.97 | 48.10 |
| | tdTomato- | SRS2549030 | 130.04 | 46.69 |
| **Cas9-Tyr-A-#2** | tdTomato+ | SRS2549033 | 135.19 | 48.53 |
| | tdTomato- | SRS2549034 | 116.56 | 41.85 |
| **Cas9-Tyr-B-#1** | tdTomato+ | SRS2549037 | 129.74 | 46.58 |
| | tdTomato- | SRS2549036 | 132.69 | 47.64 |
| **Cas9-Tyr-B-#2** | tdTomato+ | SRS2549041 | 139.00 | 49.90 |
| | tdTomato- | SRS2549028 | 134.09 | 48.14 |
| **Cas9-Tyr-C** | tdTomato+ | | 147.33 | 52.89 |
| | tdTomato- | | 147.45 | 52.94 |
| **BE3-#1** | tdTomato- | SRR8169137 | 123.84 | 44.69 |
| | tdTomato- | SRR8169136 | 128.06 | 46.21 |
| **BE3-#2** | tdTomato- | SRR8169139 | 117.97 | 42.58 |
| | tdTomato- | SRRS169138 | 128.93 | 40.53 |
| **BE3-Tyr-C-#1** | tdTomato- | SRR8169133 | 150.20 | 54.21 |
| | tdTomato- | SRRS169132 | 149.12 | 53.81 |
| **BE3-Tyr-C-#2** | tdTomato- | SRR8169135 | 15008 | 54.16 |
| | tdTomato- | SRRS169134 | 148.89 | 53.73 |
| **BE3-Tyr-D-#1** | tdTomato+ | SRRS169131 | 151.27 | 54.59 |
| | tdTomato- | SRRS169130 | 151.62 | 54.72 |
| **BE3-Tyr-D-#2** | tdTomato+ | SRK8169141 | 143.01 | 51.61 |
| | tdTomato- | SRR8169140 | 143.54 | 51.80 |
| **ABE7.10-#1** | tdTomato+ | | 133.22 | 47.83 |
| | tdTomato- | | 115.20 | 41.36 |
| **ABE7.10-#2** | tdTomato- | | 144.31 | 51.81 |
| | tdTomato- | | 143.07 | 51.36 |
| **ABE7.10-Tyr-E-#1** | tdTomato- | | 130.09 | 46.10 |
| | tdTomato- | | 148.97 | 53.48 |
| **ABE7.10-Tyr-E-#2** | tdTomato- | | 148.18 | 53.20 |
| | tdTomato- | | 133.12 | 47.79 |

The activities of Cas9, BE3 and ABE7.10 in tdTomato+ cells were confirmed by the high indel s and high SNVs ratios of the targeted sites (Figure 1C; Table 2-3).

**Table 2. WGS identification of SNVs and indels in each embryo**

| **Variants** | **Cre+** | | **Cas9+** | | | | | | | | | | | **BE3+** | | | | | | **ABE7.10+** | | | |
|---|---|---|---|---|---|---|---|---|---|---|---|---|---|---|---|---|---|---|---|---|---|---|---|
| | **#1** | **#2** | **- #1** | **- #2** | **LacZ- #1** | **LacZ- #2** | **Pde6b- #1** | **#2** | **Tyr- Pde6b- A- #1** | **Tyr- A- A- #2** | **Tyr- B- B- #1** | **Tyr- B- B- #2** | **Tyr- C** | **-#1** | **-#2** | **Tyr- C- #1** | **Tyr- C- #2** | **Tyr- D- #1** | **Tyr- D-#2** | **- #1** | **- #2** | **Tyr- E- #1** | **Tyr- E- #2** |
| **On-target mutailom** | 0 | 0 | 0 | 0 | 0 | 0 | 0 | 0 | 1 | 1 | 1 | 1 | 2 | 0 | 0 | 1 | 1 | 1 | 1 | 0 | 0 | 1 | 3 |
| **Off-target SNVs** | 2 | 25 | 22 | 3 | 8 | 2 | 0 | 0 | 22 | 10 | 5 | 33 | 31 | 277 | 137 | 320 | 355 | 332 | 277 | 1 | 1 | 17 | 21 |
| **Off-target Indels** | 0 | 3 | 0 | 1 | 0 | 1 | 0 | 0 | 0 | 0 | 2 | 0 | 4 | 1 | 0 | 1 | 4 | 1 | 0 | 1 | 1 | 3 | 2 |
| **Exon off-target SNVs** | 0 | 0 | 0 | 0 | 0 | 0 | 0 | 0 | 1 | 0 | 0 | 4 | 1 | 3 | 4 | 3 | 6 | 6 | 4 | 0 | 0 | 0 | 0 |
| **Exon off-target Indels** | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 |
| **Nonsynonymous off-target SNVs** | 0 | 0 | 0 | 0 | 0 | 0 | 0 | 0 | 0 | 0 | 0 | 2 | 1 | 2 | 2 | 0 | 4 | 4 | 2 | 0 | 0 | 0 | 0 |
| **Frameshift off-target Indels** | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 |

| | | | | | | | | | | | | | | | | | | | | | | | |
|---|---|---|---|---|---|---|---|---|---|---|---|---|---|---|---|---|---|---|---|---|---|---|---|
| ^{∗}The sgRNA for *Pde6b* has one mismatch with the C57 genome (3). so there was no on-target sites. #Two types of on-target variants, shown in Fig. S4, | | | | | | | | | | | | | | | | | | | | | | | |

**Table 3**

| **Indels** | **Mutant positions** | **Mutant** | **Mutect2 (Mut/Total reads)** | **Scalpel (Mut/Total reads)** | **Strelka (Mut/Total reads)** | **Manual realigument** |
|---|---|---|---|---|---|---|
| **Cas9-Tyr-A-#1** | ehr7:87438074 | CCAAAAGAGGG (deletion) | 1636 | 11/29 | 15/34 | 15/33 |
| **Cas9-Tyr-A-#2** | ehr7:87438083 | TCAT (insertion) | 18/37 | 13/32 | 15/35 | 13/31 |
| **Cas9-Tyr-B-#1** | ehr7:87438048 | GATAG (deletion) | 14/43 | 12/32 | 11/29 | 12/34 |
| **Cas9-Tyr-B-#2** | ehr7:87438054 | TGC (deletion) | 13/23 | 10/23 | 11/22 | 11/22 |
| **Cas9-Tyr-C** | ehr7:87498149 | CTTTGAAGGGGAA (deletion) | 44/45 | 32/32 | 45/48 | 44/45 |

| **SNVs** | **Mutant positions** | **Mutant** | **Mutect2 (Mut/Total reads)** | **Lofreq (Mut/Total reads)** | **Strelka (Mut/Total rends)** | **Manual realignment** |
|---|---|---|---|---|---|---|
| **BE3-Tyr-C-#1** | chr7:87493149 | G>A | 13/15 | 33/35 | 30/31 | 32/34 |
| **BE3-Tyr-C-#2** | chr7:87493149 | G>A | 17/36 | 17/36 | 19,40 | 22/40 |
| **BE3-Tyr-D-#1** | chr7:87492721;chr7:87492722 | C>T; C>T | 13/18; 29/19 | 15/28: | 12/28; 28/28 | 15/34; 34/34 |
| **BE3-Tyr-D-#2** | chr7:87492722 | C>T | 10/12 | 16/17 | 11/14 | 10/12 |
| **ABE7.10-Tyr-E-#1** | chr7:87438041;chr7:87438042 | G>A; G>A | 7/34; 7/34 | 7/34; 7/34 | 7/34; 7/34 | 11/39; 11/39 |
| **ABE7.10-Tyr-E-#2** | chr7:87438041;chr7:87438042; clu7:87438044:;chr7:87438039 | G>A; G>A; G>A; G>A | 20/31: 20/31; 10/32; 9/31 | 19/29 | 14/29; 17/29: 10/32; 7/29 | 24/37; 21/37; 15/37; 9/37 |

As for off-target effects, the inventors found that there were only 0-4 indels in embryos from all 12 groups (Tables 2 and 4), and none of them overlapped with predicted off-target sites (Table 5).

**Table 4**

| **Sample** | **Mutect2** | **Scalpel** | **Strelka** | **Mutect2 vs Scalpel** | **Mutect2 vs Strelka** | **Scalpel vs Strelka** | **Overlap of 3 methods** |
|---|---|---|---|---|---|---|---|
| **Cre-#1** | 107 | 11400 | 4930 | 4 | 6 | 462 | 0 |
| **Cre-#2** | 118 | 8929 | 4665 | 6 | 4 | 379 | 3 |
| **Cas9-#1** | 98 | 10854 | 4378 | 6 | 0 | 357 | 0 |
| **Cas9-#2** | 64 | 10253 | 5703 | 6 | 2 | 434 | 1 |
| **Cas9-LacZ-#1** | 131 | 11941 | 4746 | 5 | 3 | 401 | 0 |
| **Cas9-LacZ-#2** | 57 | 9394 | 5338 | 2 | 3 | 398 | 1 |
| **Cas9-Pde6b-#1** | 137 | 122S5 | 4687 | 3 | 4 | 443 | 0 |
| **Cas9-Pde6b-#2** | 125 | 12313 | 5397 | 7 | 4 | 505 | 0 |
| **Cas9-Tyr-A-#1** | 75 | 12348 | 5180 | 3 | 5 | 464 | 0 |
| **Cas9-Tyr-A-#2** | 81 | 11993 | 5480 | 3 | 4 | 471 | 0 |
| **Cas9-Tyr-B-#1** | 117 | 10659 | 4734 | 4 | 5 | 427 | 2 |
| **Cas9-Tyr-B-#2** | 70 | 9015 | 4791 | 2 | 0 | 447 | 0 |
| **Cas9-Tyr-C** | 287 | 21965 | 4539 | 13 | 14 | 828 | 4 |
| **BE3-#1** | 280 | 10654 | 3826 | 3 | 13 | 397 | 1 |
| **BE3-#2** | 269 | 10729 | 4176 | 1 | 10 | 432 | 0 |
| **BE3+Tyr-C-#1** | 289 | 14614 | 5502 | 9 | 9 | 607 | 1 |
| **BE3+Tyr-C-#2** | 259 | 14418 | 5111 | 7 | 9 | 606 | 4 |
| **BE3+Tyr-D-#1** | 273 | 14585 | 5510 | 4 | 13 | 590 | 1 |
| **BE3+Tyr-D-#2** | 268 | 12240 | 5240 | 4 | 7 | 518 | 0 |
| **ABE7.10-#1** | 284 | 53199 | 3662 | 25 | 5 | 1501 | 1 |
| **ABE7.10-#2** | 250 | 16468 | 3343 | 5 | 4 | 525 | 1 |
| **ABE7.10-Tyr-E-#1** | 283 | 90132 | 4684 | 30 | 7 | 2531 | 3 |
| **ABE7.10-Tyr-E-#2** | 238 | 32903 | 4378 | 16 | 5 | 1029 | 2 |

**Table 5**

| **Sample**-#**1** | | | | |
|---|---|---|---|---|
| | **Chr** | **Position** | **Digenome score** | **DNA sequence** |
| **Tyr-A_1 (On-target site)** | chr7 | 87438083 | 205.585443 | GCGAAGGCACCGCCCTCTTTTGG |
| **Tyr-A_2** | chr8 | 70679420 | 87.5901275 | TGGTTCATGCACCCCCCCTTAGG |
| **Tyr-A_3** | chr2 | 11906262 | 12.9829391 | catgtatagcagtgtgccagaag |
| **Tyr-A_4** | chr6 | 94012018 | 5.738594479 | CTATGGGAGGAGGTAACTAAGCG |
| **Tyr-B_1** | chr5 | 1.22E+08 | 39.98904854 | AAGAGGGCGGTGCTAAGATGGGG |
| **Tyr-B_2** | chrX | 1.12E+08 | 21.2247577 | AGGTACATAGGCTTCATATCAGG |
| **Tyr-B_3** | chr11 | 1.14E+08 | 8.274157156 | CCCATGGGGAACACTCCTGGGGG |
| **Tyr-B_4** | chr11 | 3184621 | 8.198383346 | ACAAGCAAGTGTTGGTCCATAGG |
| **Tyr-B_5** | chr11 | 1.14E+08 | 6.681354096 | CCCATGGGGAACACTCCTGGGGG |
| **Tyr-B_6** | chrX | 87640980 | 5.514465963 | CAAAAGGAGCAATTTCCAATAGG |
| **Tyr-B_7 (On-target site)** | chr7 | 87438053 | 4.826363636 | CCGATAGGTGCATTGGCTTCTGG |
| **Tyr-B_8** | chr1 | 23481074 | 4.209876693 | ATATAAGTTAACATCCCAAAAGG |
| **Tyr-⁻B_9** | chr11 | 95292492 | 3.644424083 | TATTGGGTGTCATCTCTTTCTCC |
| **Tyr-B_10** | chr1 | 1.28E+08 | 3.544329556 | CCCAAGACATGCACACCGATAGG |
| **Tyr-B_11** | chr6 | 68111031 | 2.614919835 | caagaCATAAAACATACCTAAAg |
| **LacZ_1** | chr2 | 32395622 | 43.46541216 | TTCGGCTTCGGGGCGGGGTCAAG |
| **Lac-Z_2** | chr13 | 54153138 | 37.98678846 | TAATGGTGCTGACTGCTATGAGG |
| **Pde6b_1** | chr10 | 16088519 | 65.24995196 | ATTACAATTAtttatgcctatag |
| **Pde6b_2** | chr1 | 88276189 | 5.989287063 | CTACTGCATGTTAGGAAAGGCCG |

| **Sample**-#**2** | | | | |
|---|---|---|---|---|
| | **Chr** | **Position** | **Digenome score** | **DNA sequence** |
| **Tyr-A_1** | chr8 | 70679420 | 100.166815 | TGGTTCATGCACCCCCCCTTAGG |
| **Tyr-A_2 (On-target site)** | chr7 | 87438083 | 80.04553734 | GCGAAGGCACCGCCCTCTTTTGG |
| **Tyr-A_3** | chr2 | 32395622 | 52.38775481 | AGAGGOCGGGGCCTTATAGTGGG |
| **Tyr-A_4** | chr10 | 16088519 | 48.26614325 | catgaagccaaaacacctatagg |
| **Tyr-A_5** | chr2 | 11906264 | 20.65930936 | catgtatagcagtgtgccagaag |
| T_{y}r-A_6 | chr9 | 73142622 | 5.706386646 | tcttctggtgtgtctaaagacag |
| **Tyr-A_7** | chr6 | 94012018 | 4,735788874 | CTATGGGAGGAGGTAACTAAGCG |
| **Tyr-B_1** | chr5 | 1.22E+08 | 53.80789887 | AAGAGGGCGGTGCTAAGATGGGG |
| **Tyr-B_2 (On-target site)** | chr7 | 87438053 | 48.19727891 | CCGATAGGTGCATTGGCTTCTGG |
| **Tyr-B**_**3** | chr11 | 1.14E+08 | 12.7891659 | CCCATGGGGAACACTCCTGGGGG |
| **Tyr-B**_**4** | chrX | 1.12E+08 | 8.13690641 | AAAAAGGGTTTAGTTCCTATAGG |
| **Tyr-B_5** | chr11 | 3184621 | 7.883665333 | ACAAGCAAGTGTTGGTCCATAGG |
| **Tyr-B_6** | chr16 | 24592641 | 7.196S63075 | CTATAGGCTTTGAACTGTCAGGG |
| **Tyr-B_7** | chr1 | 23481074 | 4.891318316 | ATATAAGTTAACATCCCAAAAGG |
| **Tyr-B**_**8** | chr15 | 88729863 | 2.8493S6317 | ATTCGGGCACAGCACGCAATCCG |
| **LacZ_1** | chr13 | 54153138 | 18.86615566 | TAATGGTGCTGACTGCTATGAGG |
| **LacZ_2** | chr17 | 57065755 | 4.891340168 | AGAGGGTGTTGCCTTCCCACGGG |
| **Pde6b_1** | chr4 | 69960267 | 7.637319157 | ACCTTTGGGTCCTGGGAAGGATG |

For all Cas9-edited embryos, there were no significant differences in SNVs between the different Cas9 groups (an average of 12 SNVs per embryo), and there was no significant difference compared with the "Cre" group (an average of 14 SNVs per embryo) (Table 2).

The SNVs detected in the samples treated with Cre or Cas9 may be caused by spontaneous mutations during genome replication during development. This is because the number of SNV detected herein is within the range of simulated spontaneous mutations, and the adjacent sequence showed no sequence similarity with the target site (Ref. 12).

Surprisingly, the inventors found an average of 283 SNV/embryos in embryos edited by BE3, which was at least 20 times higher than the levels observed in embryos treated with Cre or Cas9 (Figure 2A and Table 2). In contrast, ABE7.10 only produced 10 SNV/embryo on average, and the frequency was close to the spontaneous mutation rate (Figure 2A and Table 2).The inventors further compared the off-target sites identified in the "BE3 only" group with the off-target sites in BE3-Tyr-C or BE3-Tyr-D, and found that the presence of sgRNA would not induce higher SNVs (P= 0.21, Kruskal-Wallis test).In addition, these mutations were specifically identified in tdTomato+ cells instead of tdTomato- cells (see Methods, Table 6).

**Table 6**

| **SNVs** | **Mutect2** | **Lofreq** | **Strelka** | **Mutect Lofre 2 vs q** | | **Mutect2 Strelk vs a** | | **Lofreq vs Strelka** | | **Overlap of 3 methods** |
|---|---|---|---|---|---|---|---|---|---|---|
| **Cre-#1** | 527 | 66 | 865 | 4 | | 21 | | 8 | | 3 |
| **Cre-#2** | 379 | 109 | 1494 | 14 | | 42 | | 48 | | 12 |
| **Cas9-#1** | 420 | 146 | 1161 | 7 | | 29 | | 48 | | 7 |
| **Cas9-#2** | 416 | 107 | 1276 | 13 | | 38 | | 56 | | 8 |
| **Cas9-LacZ-#1** | 634 | 80 | 1111 | 3 | | 30 | | 17 | | 1 |
| **Cas9-LacZ-#2** | 604 | 68 | 1349 | 8 | | 25 | | 49 | | 6 |
| **Cas9-Pde6b-#1** | 549 | 51 | 633 | 5 | | 21 | | 3 | | 0 |
| **Cas9-Pde6b-#2** | 273 | 65 | 751 | 3 | | 38 | | **2** | | 0 |
| **Cas9-Tyr-A-#1** | 3781 | 160 | 2057 | 47 | | 374 | | 104 | | 36 |
| **Cas9-Tyr-A-#2** | 230 | 68 | 778 | 9 | | 16 | | 25 | | 8 |
| **Cas9-Tyr-B-#1** | 549 | 91 | 1009 | 14 | | 35 | | 38 | | 13 |
| **Cas9-Tyr-B-#2** | 1421 | 100 | 1391 | 16 | | 106 | | 51 | | 14 |
| **BE3-#1** | 953 | 66 | 722 | 17 | | 34 | | 20 | | 15 |
| **BE3-#2** | 968 | 75 | 807 | 23 | | 43 | | 24 | | 19 |
| **BE3-Tyr-C-#1** | 602 | 106 | 1059 | 18 | | 43 | | 32 | | 12 |
| **BE3-Tyr-C-#2** | 671 | 102 | 1019 | 24 | | 42 | | 35 | | 19 |
| **BE3-Tyr-D-#1** | 667 | 136 | 1128 | 33 | | 58 | | 55 | | 30 |
| **BE3-Tyr-D-#2** | 1261 | 64 | 1526 | 13 | | 67 | | 20 | | 7 |
| | | | | | | | | | | |

| **Indels** | **Mutect2** | **Sc alpel** | **Str elka** | **M S utcct2 vs calpel** | **Mutect2 vs Strelka** | | **S calpel Strelka** | **vs** | **Overl met ap of 3 hods** | |
|---|---|---|---|---|---|---|---|---|---|---|
| **Cre-#1** | 134 | 1 2372 | 4 380 | 1 | 383 | | 428 | | 3 | |
| **Cre-#2** | 125 | 9368 | 5 162 | **2** | 7 | | 6 | | 0 | |
| **Cas9-#1** | 177 | 1 0771 | 4-342 | 1.4 | 4 | | 393 | | 1 | |
| **Cas9-#2** | 83 | 9532 | 3 975 | 11 | 6 | | 394 | | **2** | |
| **Cas9-LacZ-#1** | 108 | 1 0849 | 4 097 | 0 | 3 | | 342 | | 0 | |
| **Cas9-LacZ-#2** | 68 | 1 0438 | 3 SS6 | 3 | **5** | | 317 | | 1 | |
| **Cas9Pde6b #1** | 255 | 1115 | 3 335 | 8 | 7 | | 256 | | 0 | |
| **Cas9-Pde6b-#2** | 215 | 3124 | 3 079 | 7 | 6 | | 255 | | 0 | |
| **Cas9-Tyr-A-#1** | 85 | 1 0913 | 4 795 | 5 | 8 | | 371 | | 4 | |
| **Cas9-Tyr-A-#2** | 78 | 8477 | 3 953 | 4 | 2 | | 459 | | 1 | |
| **Cas9-Tyr-B-#1** | 128 | 1 2457 | 4 965 | **5** | **5** | | 405 | | 2 | |
| **Cas9-Tyr-B-#2** | 79 | 1 0925 | 4 751 | 4 | **5** | | 387 | | 1 | |
| **BE3-#1** | 279 | 1 1847 | 4 127 | 7 | 4 | | 400 | | 1 | |
| **BE3-#2** | 280 | 1 2215 | 4 434 | 4 | **2** | | 440 | | 1 | |
| **BE3-Tyr-C-#1** | 240 | 1 4395 | 5 223 | 4 | 10 | | 545 | | 1 | |
| **BE3-Tyr-C-#2** | 264 | 1 5901 | 5 518 | 5 | 7 | | 617 | | 0 | |
| **BE3-Tyr-D-#1** | 291 | 1 4952 | 5 487 | 2 | 8 | | 606 | | 1 | |
| **BE3-Tyr-D-#2** | 263 | 1 2703 | 5 431 | 4 | 6 | | 517 | | 1 | |

The off-targets detected in the E3 samples were not duplicated in each group, and were randomly distributed throughout the genome. The inventors then compared these off-target mutations with all potential off-target sites predicted by Cas-OFFinder and CRISPROR softwares. Not surprisingly, these two prediction tools predicted a large number of off-target sites, but they did not appear in the SNVs detected by the inventors. In addition, there is no sequence similarity between the adjacent sequence of the identified SNVs and the BE3 sgRNA target sites, and the site with the most predicted off-target points is similar to the target site BE3 sequence. It is worth noting that although the SNV produced by BE3 editing is unique, the mutation type is consistent with the mutation type of APOBECl.

It is noted that more than 90% of the SNVs identified in the BE3 edited cells were mutations from G to A or from C to T, and no mutation preference was observed in Cre-, Cas9- or ABE7.10-treated cells (Figure 2B and C, Figure 15).Such mutation preference is the same as that of APOBEC1 itself (Reference 13), indicating that these mutations are not spontaneous, but induced by BE3 editing. Previous studies have shown that several members of the APOBEC family (including APOBEC1) require the presence of single-stranded DNA (references 14-16).The inventor's analysis also showed that BE3-induced SNV was significantly enriched in the transcribed region (Figure 3A), especially in genes with high expression (Figure 3B). Interestingly, none of the off-target sites were shared among different BE3 edited embryos or overlapped with the predicted off-target sites (Figure 3C and D).

It is reported that the combinability of DNA is related to the efficiency of gene editing. Therefore, the inventors evaluated the ATAC-seq data set from mouse embryonic cells in the Cistrome database to determine whether off-target sites are enriched in open chromatin regions. In fact, in the E8.5 embryos with mixed C57BL6/DBA2 background and the four high-quality data sets of Cistrome database, off-target sites were significantly enriched in regions with higher binding (Figure 16).

In addition, no sequence similarity was observed between off-target and target sites, and off-target sites predicted by computer showed high sequence similarity with the targeted sites of BE3. Therefore, BE3 off-target SNVs arc sgRNA-independent and may be caused by overexpression of APOBEC1.

Among the 1698 SNVs induced by BE3, 26 were located on exons, and 14 of them caused non-synonymous changes. The inventors successfully amplified 20 SNVs by PCR, and confirmed their existence by Sanger sequencing (Table 7).

**Table 7**

| **Mutant** | | **Type** | **Gene** | **Alt reads** | **Ref reads** | **Alt reads** | **Ref reads** | **Allele frequency** | **dbSNP** | **Repeats** | **PCR** | **Sanger sequeuce** |
|---|---|---|---|---|---|---|---|---|---|---|---|---|
| **BE3-#1** | | | | | | | | | | | | |
| chr2 | p.V2987M/c.119964795G>A | exonic | Mga | 11 | 20 | 0 | 39 | 35.48% | | | Y | Y |
| chr2 | p.E419N/c.140158610C>T | exonic | Esf1 | 21 | 8 | 0 | 28 | 72.41% | | | Y | Y |
| chr4 | p.L376L/c.128589747G>A | exonic | Zscan20 | 18 | 20 | 0 | 44 | 47.37% | | | Y | Y |
| **BE3-#2** | | | | | | | | | | | | |
| chr15 | p.F15F/c.80091438C>T | exonic | Syngrl | 13 | 19 | 0 | 36 | 40.63% | | | N | N |
| chr19 | p.P184P/c.60758617G>C | exonic | Nanos1 | 6 | 30 | 0 | 40 | 16.67% | | | N | N |
| chr1 | p.E488K/c.140507758G>A | exonic | Kcnt2 | 8 | 31 | 0 | 28 | 20.51% | | | Y | Y |
| chr3 | p.E59K/c.96708345C>T | exonic | Nudt17 | 14 | 23 | 0 | 41 | 37.84% | | | N | N |
| **BE3-Tyr-C-#1** | | | | | | | | | | | | |
| chr11 | p.F1507F/c.110030023G>A | exonic | Abca8a | 12 | 23 | 0 | 35 | 34.29% | | | Y | Y |
| chr3 | p.F314F/c.938261C>T | exonic | Tdpoz3 | 24 | 22 | 0 | 48 | 52.17% | | Y | Y | Y |
| chr7 | p.Q21Q/c.127020229C>T | exonic | Pnt2 | 18 | 24 | 0 | 35 | 42.86% | | | Y | Y |
| **BE3-Tyr-C-#2** | | | | | | | | | | | | |
| chr10 | p.DG27N/c.45158272G>A | exonic | Prep | 22 | 21 | 0 | 49 | 51.16% | | | Y | Y |
| chr11 | p.L29L/c.35833265C>T | exonic | Rars | 17 | 23 | 0 | 49 | 42.50% | | | Y | Y |
| chr13 | p.G230G/c.63545050C>T | exonic | Ptch1 | 27 | 17 | 0 | 33 | 61.36% | | | Y | Y |
| chr13 | p.Q282X/c.104189738G>A | exonic | Trim23 | 21 | 23 | 0 | 41 | 47.73% | | | Y | Y |
| chr16 | p.E3404K/c.15809689G>A | exonic | Prkdc | 15 | 18 | 0 | 41 | 45.45% | | | Y | Y |
| chr1 | p.Q202X/c.173462096C>T | exonic | Aim2 | 18 | 33 | 0 | 33 | 35.29% | | | Y | Y |
| **BE3-Tyr-D-#1** | | | | | | | | | | | | |
| chr11 | p.F311F/c.73354687C>T | exonic | Olfr20 | 25 | 15 | 0 | 27 | 62.50% | | | Y | Y |
| chr19 | p.E33K/c.38396211G>A | exonic | Slc35g1 | 21 | 22 | 0 | 35 | 48.84% | | | N | N |
| chr1 | p.F22F/c.60094502G>A | exonic | Wdr12 | 13 | 26 | 0 | 34 | 33.33% | | Y | Y | Y |
| chr1 | p.H346P/c.173683317A>C | exonic | Ifi208 | 7 | 58 | 1 | 48 | 10.77% | Y | Y | N | N |
| chr6 | p.D401N/c.145862884C>T | exonic | Bhlhe41 | 17 | 9 | 0 | 38 | 65.38% | | | N | N |
| chr7 | p.E421K/c.104265600C>T | exonic | Trim5 | 19 | 1 | 0 | 21 | 95.00% | | Y | Y | Y |
| **BE3-Tyr-D-#2** | | | | | | | | | | | | |
| chr14 | p.L115L/c.73563707C>T | exonic | Sucla2 | 14 | 11 | 0 | 36 | 56.00% | | | Y | Y |
| chr2 | p.E2105E/c.26460812C>T | exonic | Notch1 | 11 | **41** | 0 | 40 | 21.15% | | | Y | Y |
| chr2 | p.E872K/c.28685723G>A | exonic | Tsc1 | 9 | 29 | 0 | 33 | 23.68% | | | Y | Y |
| chr8 | p.E196K/c.11785830G>A | exonic | Arhgef7 | 14 | 22 | 0 | 37 | 38.89% | | | Y | Y |

Among the 26 SNVs, 14 caused non-synonymous changes in the encoded protein, and 2 caused premature termination in Trim23 and Aim2 genes. Trim23 encodes an E3 ubiquitin ligase whose dysfunction can lead to muscular dystrophy. Previous studies reported that the Aim2 gene plays an important role in innate immunity and is the basis against viral infections. The inventors also found one SNV on the proto-oncogcnc and 13 SNVs on the tumor suppressor gene, which has caused serious concern about the carcinogenic risk of BE3 editing (Figure 16). The inventors also found that one SNV is located on the proto-oncogene and 13 SNVs are located on the tumor suppressor gene, which raises concerns about the carcinogenic risk of BE3 editing. The inventor considered whether this risk can be reduced by expressing a lower amount of BE3. However, a lower amount of BE3 will gradually reduce the efficiency of target site editing (Table 8).

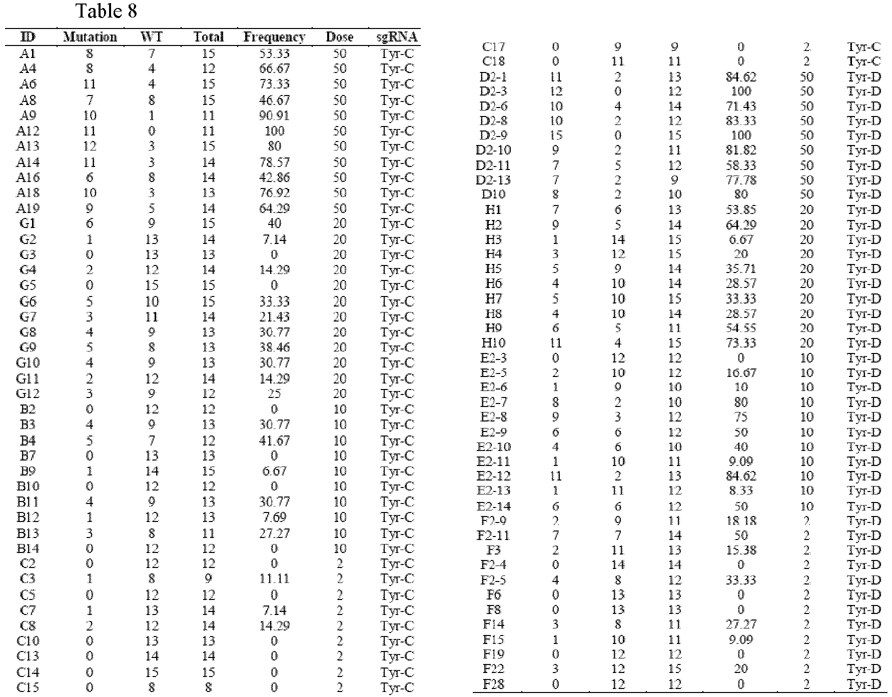

A major advantage of the method of the present disclosure is that edited and unedited cells can be compared in one animal, eliminating the difference in genetic background.The results about the comparison of edited and unedited animals in previous studies were unreliable due to differences in genetic background. In fact, the inventors also applied this method to a published data set and found that there are an average of about 1000 SNVs and about 100 indels in CRISPR/Cas9 edited and unedited mice. Based on such discovery, the inventors believe that the differences between siblings are due to genetic variation rather than the result of CRISPR/Cas9 editing. In addition, when comparing the sequences between any two different embryos, more SNVs (3706±5232) and indels (583±762) (n=18pairs) were found because the embryos used were not from the same parents.These results indicate that, even if the mice have the same parents, it is difficult to find a complete blank control for the off-target analysis to compare the edited mice with the unedited mice, due to the large amount of genetic variation among the mice.

In sum, the present disclosure proves the advantage of GOTI in studying off-target effects caused by gene editing, that is, using the daughter cells of the same embryo to perform whole-genome sequencing. The inventors also found that undesirable off-target mutations caused by CRISPR/cas9-mediated gene editing are rare in mouse embryos. This is supported by the results of previous studies that *in vivo* editing based on CRISPR/Cas9 will not cause significant SNVs and indels. However, most deletions or most chromosomal translocations reported in other studies cannot be ruled out. In contrast, the present disclosure discovers many new SNVs caused by BE3 editing, which improves the safety of base editing in therapeutic applications.

The inventors found that BE3 induced many new SNVs, which was not reported in previous studies. A possible explanation is that in the present disclosure, GOTI can detect cell populations from a single gene-edited blastomere, while previous studies used a large number of cell pools, in which editing is different, and random off-target signal is lost due to population average.Unlike BE3, ABE7.10 induced no increase in SNV, which may be due to the lack of DNA binding ability of TadA (Ref. 17).The off-target effect of BE3 may be solved by reducing the DNA binding capacity of APOBEC1 or using different forms of cytosine deaminase.In short, GOTI avoids interference of SNP among different individuals and is used to examine the off-target effects of various gene editing tools.

### Example 2. The effect of APOBEC1 enzyme on off-target effects

As disclosed above, the single-base editing tool BE3 will cause a large number of single-nucleotide off-target variations (SNV).The inventors expect that these off-target variations are caused by the overexpression of APOBEC1 and its binding to single-stranded DNA (ssDNA). However, single-base gene editing tools (BEs) have been widely used in single-base mutation research and have the potential to correct pathogenic mutations. In this example, the inventors tested the possibility of solving the off-target problem of BE3, to specifically correct the disease-related target Cs. The wild-type APOBEC1 protein sequence is shown in SEQ IDNO:1.

The BE2 system constructed for off-target evaluation of BE3 is shown in Figure 4a, which includes Apobec1, Sp nCas9 enzyme, and UGI enzyme linked through 16AA (SGSETPGTSESATPES (SEQ ID NO: 38)) and 4AA (SGGS (SEQ ID NO: 39)) peptides.

The inventors first reduced the amount of BE3mRNA injected into the embryo, and applied GOTI to detect off-target variants.As the injection amount of BE3 decreased, the efficiency of gene editing at the targeted site was correspondingly reduced (Figure 4b).Howcvcr, the number of off-target SNVs did not decrease significantly (Figure 4c).

As an alternative method, the ssDNA binding domain on Apobec1 protein was mutated to detect whether it can reduce the off-target activity of APOBEC1.The inventors mutated the corresponding amino acid positions of the corresponding BE3 based on the previous research, and used the GOTI method to evaluate their effects on the targeting efficiency and off-target effects (Figure 4a).

The inventors evaluated the efficiency of gene editing Tyr-C and Tyr-D target sites for different mutations. First, editing activity of the mutant BE3 was evaluate by use of sgRNA-C and D: BE3-W90A (at position 90 in the amino acid sequence of Apobec1 protein), BE3-W90F, BE3-R132E (at position 132 in the amino acid sequence of Apobec1 protein), BE3- R126E (at position 126 in the amino acid sequence of Apobec1 protein) and BE3-E63A (at position 63 in the amino acid sequence of Apobec1 protein). The results showed that the editing efficiency of the BE3-R126E mutation at the two target sites was not much different than that of BE3. The activity of the mutant BE3-R126E was also confirmed by the high targeting efficiency shown by WGS (Figure 4b). However, it is noted that compared with BE3, the number of off-target SNVs in R126E mutant embryos was significantly reduced, and showed no significant difference compared with "Cre only" (Figure 4c). In addition, there was not much difference between the two embryos treated with R126E. The amount of detected SNVs was close to the spontaneous mutation rate, and there was no overlap of SNV with predicted potential off-target sites, indicating that mutation from arginine to glutamic acid at position 126 of Apobec1 can significantly reduce BE3-induced off-target SNVs.

Thereofore, the present inventors revealed for the first time a solution to solve the off-target effect induced by BE3 by mutating APOBEC1, such as R126E.

The modularity established in the present disclosure indicates that GOTI is a further solution for other mutant versions of APOBEC1 or a newly engineered cytidine deaminase.

### Example 3. Research on mutation optimization

First, the present inventors injected different amounts of BE3 mRNA (50ng/µl and 10ng/µl) together with sgRNA-Tyr-C or sgRNA-Tyr-D into embryos, and evaluated the targeting efficiency by single-cell Sanger sequencing.

It is found that using a smaller amount of BE3 can significantly reduce the targeting efficiency (72.6±5.3%, 50ng/µl; 12.6±2.9%, 10ng/µl)

Then whole-genome off-target assessment was performed by GOTI method. Genome-wide off-target analysis by two-cell embryo injection (GOTI) detected off-target variants on BE3-Tyr-D-treated embryos, and it is found that the number of off-target SNVs of BE3mRNA in two different level (injected with 50ng/nl and 10ng/nl) did not change.

Then the inventors detected whether a point mutation at the DNA binding domain of APOBEC1 would reduce the off-target rate of BE3. Based on the DNA binding domain identified in previous studies, the inventors introduced various point mutations into the putative DNA binding domain of APOBEC1 in the BE3 system, and evaluated their cffccts on on-targct efficiency and off-target rate (Figure 5a). For E63A, R126E, and R132E, the base editing efficiency of BE3 was evaluated in targeted base editing at two sites of the Tyr gene, wherein the 2-cell mouse embryos contained corresponding sgRNA Tyr-C and Tyr-D (Figure 5b). It is found that, compared with wild-type BE3, the editing efficiency of BE3-E63A or BE3-R132E on Tyr was significantly reduced, while BE3-R126E maintained high editing efficiency at both target sites.The inventors further confirmed that the DNA targeting activity of BE3-R126E is similar to BE3 at the other three sites in HEK293T cells. Interestingly, the editing window of BE3-R126E has shrunk.

Then GOTI was used to evaluate on-target efficiency and off-target frequency of BE3-R126E in the three groups with or without sgRNA (BE3-R126E, BE3-R126E-Tyr-C and BE3-R126E-Tyr-D), BE3-W90Y+R126E(YE1)-Tyr-C and BE3-W90F+R126E(FE1)-Tyr-C. The on-target efficiency was confirmed by whole genome sequencing (Figure 5c). It is noted that the amount of off-target SNVs in embryos treated with BE3-R126E and BE3-W90Y+R126E (YE1) was significantly reduced from 283±2 (n=6) in embryos treated with wild-type BE3 to 24±8, which is closed to spontaneous mutation (Figure 5d). In addition, no mutational deviation was observed and no SNV overlapped with the predicted off-target site, indicating that the off-target SNV induced by BE3R126E does not exist.

The inventors further detected the off-target effects in BE3-W90Y+R126E (YE1) and BE3-R126E on 293T cells. It was found that BE3-R126E can significantly reduce RNA off-target. BE3-W90Y+R126E(YE1) can completely eliminate RNA off-target (Figure 5e).

In Figure 5d-e, BE3 (hA3A) is a new BE3 editing tool constructed using human APOBECA3A (human APOBECA3A) instead of apobec1 on BE3. BE3 (hA3AY130F) contains mutation Y130F in human APOBECA3A. It can be observed that this mutation significantly reduces the number of off-target SNVs.

In conclusion, by applying the GOTI method to assess the amount of off-target SNVs, it can be proved that by mutating the putative ssDNA binding domain of the deaminase of the base editor can eliminate the off-target effect of the cytosine base editor at the DNA and RNA levels.

The results indicate that a base editor can be designed as an effective and safe tool for gene editing and therapeutic applications.

Each reference provided herein is incorporated by reference to the same extent as if each reference was individually incorporated by reference.In addition, it should be understood that based on the above teaching content of the disclosure, those skilled in the art can practice various changes or modifications to the disclosure, and these equivalent forms also fall within the scope of the appended claims.

### References:

1. G. J. Knott, J. A. Doudna, CRISPR-Cas guides the future of genetic engineering. Science 361, 866-869 (2018).
2. S. Q. Tsai, J. K. Joung, Defining and improving the genome-wide specificities of CRISPR-Cas9 nucleases. Nat Rev Genet 17, 300-312 (2016).
3. C. R. Lazzarotto et al., Defining CRISPR-Cas9 genome-wide nuclease activities with CIRCLE-seq, Nat Protoc 13, 2615-2642 (2018).
4. K. R. Anderson et al., CRISPR off-target analysis in genetically engineered rats and mice.Nat Methods 15, 512-514 (2018).
5. D. Kim et al., Genome-wide target specificities of CRISPR RNA-guided programmable deaminases. Nat Biotechnol 35, 475-480 (2017).
6. T. I Cornu, C. Mussolino, T. Cathomen. Refining strategies to translate genome editing to the clinic. Nature Medicine 23, 415-423 (2017).
7. H. A. Rees, D. R. Liu, Base editing: precision chemistry on the genome and transcriptome cf living cells. Nat Rev Genet, (2018).
8. N. M. Gaudelli et al., Programmable base editing of A*T to G*C in genomic DNA without DNA cleavage. Nature 551, 464-471 (2017).
9. L. Madisen et at., A robust and high-throughput Cre reporting and characterization system for the whole mouse brain. Nat Neurosci 13, 133-140 (2010).
10. L. Wang et al., CRISPR-Cas9-mediated genome editing in one blastomere of two-cell embryos reveals a novel Tet3 function in regulating neocortical development. Cell Res 27, 815-829 (2017).
11. K. Kim et al., Highly efficient RNA-guided base editing in mouse embryos. Nat Biotechnol 35, 435-437 (2017).
12. J. W. Drake, B. Charlesworth, D. Charlesworth, J. F. Crow, Rates of spontaneous mutation. Genetics 148, 1667-1686 (1998).
13. A. C. Komor, Y. B. Kim, M. S. Packer, J. A. Zuris, D. R. Liu, Programmable editing of a target base in genomic DNA without double-stranded DNA cleavage. Nature 533, 420-424 (2016).
14. R. S Harris, S. K. Petersen-Mahrt, M. S. Neuberger, RNA editing enzyme APOBEC1 and some of its homologs can act as DNA mutators. Mol Cell 10, 1247-1253 (2002)
15. S. Rebhandl, M. Huemer, R. Greil, R. Geisberger, AID/APOBEC deaminases and cancer. Oncoscience 2, 320-333 (2015).
16. L. B. Alexandrov et al., Signatures of mutational processes in human cancer. Nature 500, 415-421 (2013).
17. H. C. Losey, A. J. Ruthenburg, G. L. Verdine, Crystal structure of Staphylococcus aureus tRNA adenosine deaminase TadA in complex with RNA. Nat Struct Mol Biol 13, 153-159 (20C6).
18. S Jin et al., Cytosine, but not adenine, base editors induce genome-wide off-target mutations in rice. Science, in press (2019).
19. Y. B. Kim et al., Increasing the genome-targeting scope and precision ot base editing with engineered Cas9-cytidine deaminase fusions. Nat Biotechno/ 35, 371-376 (2017).
20. X. Wang et al., Efficient base editing in methylated regions with a human APOBEC3A-Cas9 fusion. Nat Biotechnol 36, 946-949 (2018).
21. J. M. Gehrke et al., An APOBEC3A-Cas9 base editor with minimized bystander and off-target activities. Nat Biotechnol 36, 977-982 (2018).

## Claims

1. A method for reducing the off-target effect of a single-base editor, comprising: modifying the cytosine deaminase in a single-base editor system to weaken its binding to DNA.

2. The method according to claim 1, wherein, modifying the cytosine deaminase is to modify the DNA binding region of the cytosine deaminase; preferably, the DNA binding region is a domain thereof that binds to DNA.

3. The method according to claim 2, wherein, the modification comprises, but is not limited to: gene mutation, targeted blocking, interference.

4. The method according to claim 1, wherein, the single-base editor system is a BE3 gene editor system, or
the DNA is single-stranded DNA or double-stranded DNA.

5. The method according to claim 1, wherein, the cytosine deaminase comprising but is not limited to an enzyme selected from the group consisting of: AID, APOBEC3G, APOBEC1, APOBECA3A, CDA1.

6. The method according to claim 5, wherein, the cytosine deaminase is APOBEC1; preferably, modifying the cytosine deaminase is to modify the amino acid at position 126 of the enzyme; more preferably, to modify R126 of the enzyme to E.

7. The method according to claim 6, wherein, modifying APOBEC1 comprising: modifying the amino acid at position 90 of the APOBEC1 enzyme; preferably, modifying the amino acid at position 90 to Y.

8. The method according to claim 5, wherein, the cytosine deaminase is APOBECA3A, and modifying the cytosine deaminase is to modify the amino acid at position 130 of the enzyme; more preferably, the enzyme is modified to alter Y at position 130 to F

9. A mutant of cytosine deaminase, wherein the DNA binding region of the cytosine deaminase is modified to weaken its binding to DNA, such as single-stranded DNA; preferably, the cytosine deaminase comprises but is not limited to an enzyme selected from the group consisting of: AID, APOBEC3G, APOBEC1, APOBECA3A, CDA1.

10. The mutant according to claim 9, wherein, the enzyme is APOBEC1; preferably, the domain is modified at or near position 126 of the domain; more preferably, the domain is modified to alter R at position 126 to E.

11. The mutant according to claim 9, wherein, APOBEC1 is further modified at the 90th amino acid of the enzyme; preferably, the enzyme is modified to alter the amino acid at position 90 to Y.

12. The mutant according to claim 9, wherein, the enzyme is APOBECA3A, and the enzyme is modified at or near position 130 of the enzyme; more preferably, the enzyme is modified to alter Y at position 130 to F.

13. An isolated polynucleotide, wherein the polynucleotide encodes the mutant according to any of claims 9-12.

14. A single-base editor, comprising a mutant of the cytosine deaminase according to any of claims 9-12; preferably, the editor is a BE3 single-base editor.

15. Use of the mutant of cytosine deaminase according to any of claims 9-13 in gene editing based on a single-base editor system to reduce the off-target effect of the gene editor.

16. A method for screening a substance for reducing the off-target effect of a single-base editor, comprising:
(1) treating a system with a candidate substance, the system containing interaction between a cytosine deaminase or its DNA binding domain and DNA; and
(2) detecting the interaction between the cytosine deaminase or its DNA binding domain and DNA in the system; wherein, if the candidate substance inhibits, blocks or down-regulates the interaction between the cytosinc deaminase or its DNA binding domain and DNA, the candidate substance is useful for reducing the off-target effect of the gene editor.

17. A method for analyzing the on-target effect of gene editing or the on-target effect of a single-base gene editing tool, the method comprising:
(1) obtaining a n-cell stage embryo, subjecting one to n-1 cells thereof to gene editing, wherein n is a positive integer from 2 to 10;
(2) observing or detecting the occurrence of gene editing in the downstream development stages of the embryo.

18. The method according to claim 17, wherein, in step (1), n is a positive integer of 2 to 8,2 to 6 or 2 to 4; preferably, n is 2.

19. The method according to claim 17, wherein, the method is an *in vitro* cultivation method or an *in vivo* cultivation method.

20. The method according to claim 17, wherein, in step (2), the downstream development stage of the embryo is from gastrulation stage of the embryo to prenatal stage, or from embryo implantation into a uterus to prenatal stage *in vivo.*

21. The method according to claim 17, wherein, the embryo is a mouse embryo, and the downstream development stage of the embryo is the 8th to 20th day of embryonic development (E8-E20 stage), preferably is the 9.5th to 18.5th day of embryonic development (E9.5-E18.5 stage), more preferably is the 12th to 16th day of embryonic development (E11-E16 stage, such as E14.5).

22. The method according to claim 17, wherein, during the cleavage stage of the embryo, the gene-edited blastomere and the unedited blastomere of the embryo is separated and transplanted into recipients to develop separate adults.

23. The method according to claim 22, wherein, the gene-edited blastomere and the unedited blastomere form separate embryos, which are transplanted to different recipients or the same recipient, or used to establish embryonic stem cell lines *in vitro.*

24. The method according to claim 17, wherein, the gene editing comprises (but are not limited to): CRISPR-mediated gene editing, Base Editor (base editor)-mediated gene editing, Cre/loxP-mediated gene editing, Prime editor.

25. The method according to claim 24, wherein, the CRISPR-mediated gene editing comprises (but not limited to): CRISPR/Cas9-mediated gene editing, CRISPR/Cas9n-mediated gene editing, CRISPR/Cas13 (such as CRISPR/Cas13a, CRISPR/Cas13d)-mediated gene editing, CRISPR/CasRx-mediated gene editing.

26. The method according to claim 24, wherein, the Base Editor comprises: BE1, BE2, BE3, BE4, or BE4-Max.

27. The method according to claim 24, wherein, the adenine base editor comprises: ABE7.10, ABE6.3, ABE7.8, ABE7.9, Prime Editing.

28. The method according to claim 17, wherein, step (1) comprises: introducing an enzyme (such as Cas mRNA, Cre mRNA) for cutting a nucleic acid (such as DNA) target site together with a corresponding guide sequence (such as sgRNA) into one of the cells, and performing gene editing.

29. The method according to claim 28, wherein, the enzyme for cutting a nucleic acid (such as DNA) target site is selected from but not limited to the group consisting of: Cas9, Cas9n, Cas13a, CasRx, BE1, BE2, BE3, BE4, ABE7.10, ABE 6.3, ABE 7.8, ABE 7.9.

30. The method according to claim 17, wherein, in step (1), a detectable marker is used to label the gene editing, and the gene editing is performed on 1 to n-1 of the cells and labeled by the detectable marker.

31. The method according to claim 30, wherein, the detectable marker includes but is not limited to: a dye marker, a fluorescent signal molecule, a reporter gene; more preferably, the detectable marker is (but not limited to) tdTomato, EGFP, mCherry, GFP, dsred.

32. The method according to claim 17, wherein, in step (2), observing the occurrence of gene editing comprises:
sorting cells that have undergone gene editing and cells that have not undergone gene editing;
analyzing by sequencing;
analyzing through a single nucleotide variation analysis tool and/or a indel analysis tool;
comparing edited cells with unedited cells to identify on-target effects or off-target effects, including detection of SNVs and indels.

33. The method according to claim 32, wherein, the single nucleotide variation analysis tool comprises but is not limited to: Mutect2, Lofreq and Strelka or a combination thereof; or the indel analysis tool comprises but is not limited to: Mutect2, Scalpel, Strelka or a combination thereof.

34. The method according to claim 17, wherein, the embryo is derived from a mammal, including but not limited to a non-human mammal.
